(19) Europäisches Patentamt
European Patent Office
Office européen des brevets

(11) **EP 3 649 866 B1**

(12) **EUROPEAN PATENT SPECIFICATION**

(45) Date of publication and mention
of the grant of the patent:
**13.11.2024 Bulletin 2024/46**

(21) Application number: **19204208.3**

(22) Date of filing: **18.10.2019**

(51) International Patent Classification (IPC):
*A23C 9/12* (2006.01)    *A23C 9/123* (2006.01)
*A23C 9/158* (2006.01)    *A23L 3/3571* (2006.01)
*A61P 31/04* (2006.01)    *C07K 14/335* (2006.01)
*C12N 1/20* (2006.01)    *C12R 1/225* (2006.01)
*A23C 11/10* (2021.01)    *C12R 1/01* (2006.01)
*A61K 35/747* (2015.01)    *A61K 35/00* (2006.01)
*A61K 35/744* (2015.01)

(52) Cooperative Patent Classification (CPC):
(C-Sets available)
**A23C 11/10; A23C 9/1234; A23C 9/1236;
A23C 11/106; A61K 35/744; A61K 35/747;
A61P 31/04; C12N 1/20; C12N 1/205;**
A23V 2002/00; A23V 2400/11; A61K 2035/115;
C12R 2001/01; C12R 2001/225    (Cont.)

(54) **LACTOBACILLUS ALIMENTARIUS MK426 FOR USE IN PREVENTING INFECTIONS WITH LISTERIA SPECIES, COMPOSITIONS COMPRISING SAID STRAIN AND USES THEREOF**

LACTOBACILLUS ALIMENTARIUS MK426 ZUR VERWENDUNG BEI DER PRÄVENTION VON INFEKTIONEN MIT LISTERIA-SPEZIES, DIESEN STAMM ENTHALTENDE ZUSAMMENSETZUNGEN UND DEREN VERWENDUNG

LACTOBACILLUS ALIMENTARIUS MK426 POUR UTILISATION DANS LA PRÉVENTION DES INFECTIONS PAR DES ESPÈCES DE LISTERIA, COMPOSITIONS COMPRENANT CETTE SOUCHE ET UTILISATIONS DE CELLES-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.10.2018 PT 2018115088**

(43) Date of publication of application:
**13.05.2020 Bulletin 2020/20**

(73) Proprietor: **Universidade Católica Portuguesa - UCP**
**4169-005 Porto (PT)**

(72) Inventors:
• **MAIA TEIXEIRA, PAULA CRISTINA**
**4169-005 PORTO (PT)**
• **MACIEL E SILVA, CLÁUDIA**
**4169-005 PORTO (PT)**
• **FERREIRA, VÂNIA**
**4169-005 PORTO (PT)**
• **SOUSA, SÉRGIO**
**4169-005 PORTO (PT)**
• **KOMORA DA SILVA, NÓRTON**
**169-005 PORTO (PT)**

(74) Representative: **Patentree
Edificio Net
Rua de Salazares, 842
4149-002 Porto (PT)**

(56) References cited:
**EP-A1- 0 326 062    WO-A1-2005/100614
CN-A- 104 830 731    JP-A- 2003 230 376**

• **YANXIN HU ET AL: "Novel bacteriocin produced by Lactobacillus alimentarius FM-MM 4 from a traditional Chinese fermented meat Nanx Wudl: Purification, identification and antimicrobial characteristics", FOOD CONTROL., vol. 77, 6 February 2017 (2017-02-06), GB, pages 290 - 297, XP055673273, ISSN: 0956-7135, DOI: 10.1016/j.foodcont.2017.02.007**

- JUVEN BENJAMIN J. ET AL: "Growth and Survival of Listeria monocytogenes in Vacuum-Packaged Ground Beef Inoculated with Lactobacillus alimentarius FloraCarn L-2", JOURNAL OF FOOD PROTECTION, vol. 61, no. 5, 1 May 1998 (1998-05-01), US, pages 551 - 556, XP093053129, ISSN: 0362-028X, DOI: 10.4315/0362-028X-61.5.551

(52) Cooperative Patent Classification (CPC): (Cont.)

C-Sets
**A61K 35/744, A61K 2300/00;**
**A61K 35/747, A61K 2300/00;**
A23V 2002/00, A23V 2200/10, A23V 2200/3204;
A23V 2400/11, A23V 2200/30, A23V 2200/32,
A23V 2400/41, A23V 2400/413

**Description**

**Technical field**

The present disclosure relates to new bacteria, use of the selected bacteria in a composition, food product or foodstuff comprising the selected bacteria, fermented products and a method to obtain a novel fermented dairy product or dairy alternative product, in particular coconut milk or oat yogurt-like fermented product, with probiotic activity.

The present disclosure also relates to a novel fermented dairy product or dairy alternative product, in particular dairy milk or coconut milk/oat yogurt-like comprising the bacterium selected in the present disclosure that may be used to prevent infections, preferably as a prophylactic agent, namely to counteract against *Listeria monocytogenes.*

**Background**

Fermented milks are among nature's most prominent contributions to civilization. Historically, these foods have allowed population to survive periods of starvation and were prescribed as a medicine for curing disorders of the stomach and intestine. Nowadays, fermented milk is considered one of the most popular dairy products, consumed in many societies worldwide, being recognized as a healthy food product. In fact, the manufacture of cultured dairy products represents the second most relevant fermentation industry. Fermented milk is a cultured dairy product made by the fermentation of heated milk with lactic acid bacteria, which converts lactose into lactic acid resulting in a pH reduction. The process of acidification, lowers the pH below the isoelectric point of casein (pH 4.6-4.8) promoting aggregation and precipitation of casein micelles, eliciting a fermented product with a complex and dynamic three-dimensional casein network surrounded by midsize fat and serum globules, soft solid textured gel and determines the palatability of the fermented milk, conferring its characteristic tart flavour. Nutritionally they provide a richness of essential elements to good health, namely protein, essential minerals (e.g., calcium, fulfilling the daily nutritional requirements) and vitamins (vitamin D, riboflavin, vitamin B6 and B12). Lactic acid is one of the most prominent metabolites biosynthesized in amounts over 1%, which recently demonstrated to reduce pro-inflammatory cytokine secretion of TLR-activated, bone-narrow-derived macrophages and dendritic cells in a dose-dependent manner. This popular dairy product is considered an extraordinary vehicle for the delivery of probiotics, mainly due to their good compatibility as well as the pleasant and attractive sensory features provided by these notable bacteria, attracting much attention in the food industry.

The concept behind probiotics was probably first introduced more than a century ago, when the Nobel laureate Elie Metchnikoff hypothesized that the intriguing longevity of the bulgarian peasants was correlated with the ingestion of milk fermented by "good" lactic acid-producing bacteria (LAB), namely those belonging to the genera *Lactobacillus.* Contemporary with this first report, Henry Tissiert observed that a bacterium presenting a Y-shaped morphology, denominated "bifidus", was abundant in the stools of healthy breast-fed infants, but scarce in the stools of formula-fed infants with diarrhea. These first scientific observations highlighted a beneficial role played by some intestinal bacteria to maintain or restore a healthy gut flora. According to the Food and Agriculture Organization of the United Nations (FAO) and the World Health Organization (WHO) Expert Committee, probiotics have been defined as "live microorganisms which when administered in adequate amounts confer a health benefit on the host". *Lactobacillus* and *Bifidobacterium* species, indigenous inhabitants of the gastrointestinal tract (GIT), represent the most predominantly used probiotics, although *Escherichia coli* str. Nissle 1917, *Enterococcus,* certain *Bacillus* species, and certain yeasts, namely *Saccharomyces boulardii* and *Saccharomyces cerevisiae* are used as well. It has been proposed that probiotics may positively impact human health through the improvement of digestion function and uptake of essential nutrients, modulation of the host's immune system, specifically in the small intestine, maintenance or enhancement of bowel function, promotion of host resistance to gastric and enteric bacterial pathogens and reduction of the risk of allergy. In order to perform their peculiar health activities through transient gut colonization, probiotics should exhibit some essential features, namely, to overcome the harsh gastrointestinal environment, in particular low pH adaptation (stomach) and bile tolerance (small intestine), being able to stably survive and conserve metabolic activity and the ability to adhere to mucus and epithelial cells, which may prolong their persistence in the GIT. These properties are also used as important selection criteria for potential probiotic strains, among which may also be included a lack of transmissible antibiotic resistance genes, safety for human use (being non-pathogenic and nontoxic), experimentally and clinically demonstrated health benefits and the capability to withstand harsh conditions of the technological processes.

The large set of mechanisms by which probiotic strains may exert their antagonism towards undesired enteropathogens are complex, including bacteriostatic and bactericidal activities. In this sense, among the modes of action may be cited the capability of competitive exclusion of harmful pathogens, through blocking their contact with receptors expressed on epithelial cells and subsequent downstream effects (Corr et al., 2009). This mechanism of action is also known as colonization resistance. Probiotics also appear to enhance epithelial barrier function via induction of innate immune proteins, including mucus or β-defensin production; or augmenting tight junction function. They may also promote the activation of epithelial adaptive immune responses, increasing secretion of secretory IgA into the luminal mucus layer and anti-inflammatory cytokines such as IL-10 and TGF-$\beta$, while suppressing mucosal inflammation putatively via Toll-like receptor signalling (Corr et al., 2009). These immunomodulatory effects may be exerted via dendritic cells, which display a key role in the early bacterial recognition and in modulating T cells response. Another mechanism by which probiotics may enhance their anti-infectious effects is through the modulation of the expression of virulence genes coding for virulence factors or interference with the cell membrane expression or secretion of virulence factors, namely toxins (Corr et al., 2009). Alternatively, some probiotic strains may present antagonistic activities against the signalling-regulated functional and structural detrimental effects promoted by enteropathogenic virulence factors at the host's epithelial barrier (Corr et al., 2009). Finally, some probiotics have the potential to directly exert their inhibitory activity through production of organic acids and antimicrobial factors, which may act alone or synergistically. These secreted metabolites include lactic acid, hydrogen peroxide, short-chain fatty acids, proteases directed against bacterial toxins, non-bacteriocin compounds, non-proteinaceous molecules and the renewed bacteriocins.

*In situ* bacteriocin production is regarded as a notable probiotic trait (Dobson et al., 2012). Bacteriocins are small antimicrobial peptides or proteins, ribosomally biosynthesized by one bacterium and presenting bacteriostatic or bactericidal activity primarily directed against closely related species (narrow spectrum). These promising peptides could offer an alternative to antibiotic prophylaxis/treatment with regard to infectious diseases, namely listeriosis. In fact, the first-in-animal study has been performed, demonstrating the pivotal role of the bacteriocin Abp118 in mediating the direct antagonism exerted by the probiotic *L. salivarius* UCC 118 on *Listeria monocytogenes.*

*Listeria monocytogenes* is the etiological agent of human listeriosis, a low incident but deadly disease, presenting a high fatality rate (20-30%). Listeriosis is increasing in Europe, being considered the most frequent cause of hospitalization/death due to the consumption of contaminated food. Indeed, surveillance studies and investigation of recent outbreaks have demonstrated that milk and dairy products have been associated with several outbreaks in recent years. The infectious dose of *L. monocytogenes* is considered to be between $10^6$ and $10^9$ colony forming units (CFU), but is highly dependent upon the nature of the food matrix and host susceptibility. Although it can occur in healthy individuals, newborn, pregnant women, elderly individuals and immunocompromised individuals are at higher risk for listeriosis. The bacteria present an amazing capacity to cross protective epithelial barriers, namely blood-brain, intestinal and placental. Once it infects the host, *L. monocytogenes* crosses the intestinal epithelium, via transcytosis, and rapidly spreads through the lymph or blood to the mesenteric lymph nodes, the spleen, and the liver (Cossart et al., 2018).

Plant-based yogurt-like fermented products, remains an unexplored food category. In fact, over the last decades the consumers awareness regarding nutrition and environmental issues has contributed to boost the introduction of novel products meant for the healthy/ vegetarian market niche. For example, strict vegetarians and vegans have difficult access to probiotic yogurt like products, as the basis of these mentioned products is dairy, which does not allow them the consumption. Also, numerous reports pointed out the notable health promoting effects of the plant-based food consumption, namely coconut and oat-derived products, with regard to chronic diseases, namely coronary insufficiencies, cancer, diabetes and obesity. In the study herein presented, we developed novel plant-based yogurt-like products with prophylactic potential regarding listeriosis, having oat and coconut as milk replacers.

Yanxin Hu et al "Novel bacteriocin products by Lactobacillus alimentarius FM-MM 4 from a traditional Chinese fermented meat Nanx Wudl: Purification, identification and antimicrobial characteristics" (Food Control., vol. 77, 6 Feb 2017, pages 290-297) discloses a novel bacteriocin (non-analogous to coagulin A described herein) produced by *Lactobacillus alimentarius* FM-MM4, which was isolated from Nanx Wudl, a traditional Chinese fermented meat.

Juven Benjamin J. et al "Growth and Survival of Listeria monocytogenes in Vacuum-Packaged Ground Beef Inoculated with Lactobacillus alimentarius FloraCarn L-2" (Journal of Food Protection, vol. 61, 1998, pages 551-556) discloses the commercial starter culture FloraCarn L-2 (Christian Hansen) consisting of a pure strain of *Lactobacillus alimentarius.* FloraCarn L-2 was demonstrated to have an antilisterial effect. However, *Lactobacillus alimentarius* was disclosed not to produce a bacteriocin or hydrogen peroxide, but to prevent *L. monocytogenes* growth via the production of lactic acid.

These facts are disclosed in order to illustrate the technical problem addressed by the present disclosure.

## General Description

The present disclosure relates to a new bacterium, use of the selected bacterium in a composition, food product or foodstuff comprising the selected bacterium, fermented products and a method to obtain a novel fermented dairy product or dairy alternative product, in particular milk, with probiotic activity.

The present disclosure also relates to a novel fermented dairy product or dairy alternative product, in particular dairy milk or coconut milk/oat yogurt-like product comprising the bacteria selected in the present disclosure that may be used to prevent infections, preferably as a prophylactic agent, namely, to counteract *Listeria monocytogenes.*

The whole genome of *Lactobacillus alimentarius* MK426 is also herein disclosed in the sequence listing section - SEQ ID NO 1.

The whole genome of *Pediococcus acidilactici* MK952 (not falling under the scope of protection) is also herein disclosed in the sequence listing section - SEQ ID NO 2 as an example.

An aspect of the present disclosure relates to the use of *Lactobacillus alimentarius* MK426 for fermented food products preparation.

Another aspect of present disclosure relates to a composition comprising *Lactobacillus alimentarius* MK426. Preferably a pharmaceutical or a food or food stuff composition.

In the present disclosure it is considered a dairy alternative product, namely a novel plant-based yogurt-like product with prophylactic potential regarding listeriosis, having oat or coconut as milk replacers, as well as soy milk, rice milk, almond milk, yogurt alternative, cheese alternative, frozen-yogurt alternative, ice-cream alternative, cream alternative, among others.

Another aspect of present disclosure relates to a method for obtaining a fermented dairy product comprising the step of fermentation by *Lactobacillus alimentarius* MK426, or *Pediococcus acidilactici* MK952 (not falling under the scope of protection) as an example.

The incorporation of live antilisterial bacteriocin-producing strains through the fermentation process, namely in dairy products, seems to present a benefit in counteracting this opportunistic pathogen. This disclosure relates to a novel functional probiotic-based fermented milk with *in situ* antilisterial bacteriocin delivery, which holds a promising approach for a rare but deadly disease such as listeriosis, where prophylactic chemotherapy or vaccination could not be an option. This would be of particular relevance for the elderly given the extraordinary rate at which the global population is aging, as well as the unprecedented longevity that is achieved in some parts of the world.

In an embodiment, the amount of *Lactobacillus alimentarius* MK426, vary between 0.5-2% (v/v).

In a further embodiment, the fermentation time of the method of the present disclosure varies between 140-160 min.

In an embodiment, the pH of the method of the method of the present disclosure varies between 4.4-4.6.

In an embodiment, the method of the present disclosure further comprises the addition of: carbohydrates (sucrose and or glucose), hydrocolloid (gelatin, agar agar) or skim milk powder, or mixtures thereof.

In an embodiment, the ratio between bacteria/milk varies between from 9-11 log CFU mL$^{-1}$, preferably 10.1-10.4 log CFU mL$^{-1}$.

Another aspect of the present disclosure relates to dairy product comprising a bacteriocin obtainable by the fermentation with *Lactobacillus alimentarius* MK426.

Another aspect of the present disclosure relates to dairy product for use in the prevention of listeriosis.

In an embodiment, the dairy product can be milk, cheese, yogurt, and other derivates, among others.

Another aspect of the present disclosure relates to food or pharmaceutical composition comprising the dairy product described in the present disclosure. *Lactobacillus alimentarius* MK426 with a complete genome sequence of: SEQ ID NO 1 for use in prevention of *Listeria* infection.

A preferred aspect of the invention is *Lactobacillus alimentarius* MK426 with a complete genome sequence of: SEQ ID NO 1 for use as a probiotic agent or as a nutraceutical agent.

In an embodiment the present invention discloses the use of *Lactobacillus alimentarius* MK426 for fermented food products preparation.

In a further embodiment, the present invention comprises a composition with *Lactobacillus alimentarius* MK426.

In a further embodiment, the present invention comprises a composition according to claim 2 for use as a probiotic.

In a further embodiment, the present invention comprises a composition according to claim 1 for use in the prevention of *Listeria* infection.

In a further embodiment, the present invention comprises a composition wherein the composition is a food composition or a pharmaceutical composition.

In a further embodiment, the present invention comprises a composition wherein the composition is dried, lyophilized or in paste form.

In a further embodiment, the present invention comprises a composition further comprising carbohydrates, hydrocolloids, skim milk powders, stabilizers, emulsifiers, preservatives, buffers, or mixtures thereof.

In a further embodiment, the present invention comprises a composition wherein the number of bacteria varies from 9-12 CFU mL$^{-1}$, preferably from 10.1-10.4 log CFU mL$^{-1}$.

In a further embodiment, the present invention comprises a food product or foodstuff comprising *Lactobacillus alimentarius* MK426.

In a further embodiment, the present invention comprises a food product or foodstuff, wherein the food product or foodstuff is a diary product or dairy alternative product comprising a bacteriocin obtainable by the fermentation with *Lactobacillus alimentarius* MK426.

In a further embodiment, the present invention comprises a food product or foodstuff, wherein the product is milk, cheese, yogurt, frozen yogurt, or ice-cream, an oat derivate, a coconut derivate, a soy derivate or mixtures thereof.

A cereal-based culture medium for the production of *Lactobacillus alimentarius* is disclosed (not falling under the scope of protection).

In an embodiment the present invention relates to a method for obtaining a dairy product or dairy alternative product of the present invention comprising the steps of:

fermenting the dairy product or dairy alternative product by *Lactobacillus alimentarius* M K426;
in particular wherein the amount of *Lactobacillus alimentarius* MK426 vary from 0.5 to 2% (v/v);
in particular wherein the fermentation time varies from 140 to 160 min.
in particular wherein the pH varies from 4.4 to 4.6.

**Brief Description of the Drawings**

The following figures provide preferred embodiments for illustrating the description and should not be seen as limiting the scope of invention.

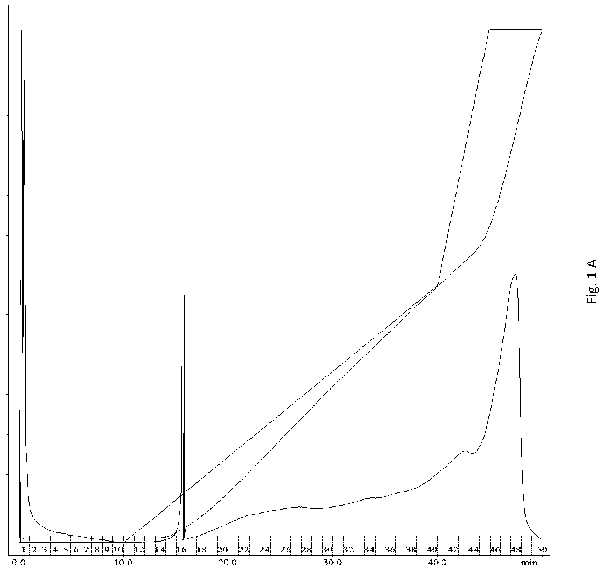

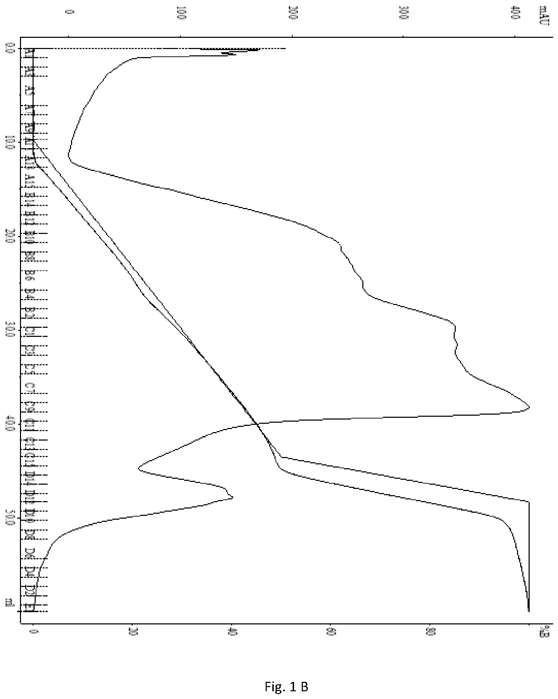

Figure 1. Ion exchange chromatograms of the bacteriocins secreted by *Lactobacillus alimentarius* MK426 (A) and *Pediococcus acidilactici* MK952 (B), respectively. Proteins were eluted with an ascending and linear gradient of NaCl (0 to 1 M) in 50 mM acetate buffer pH 5.6, at a flow rate of 1 mL/min.

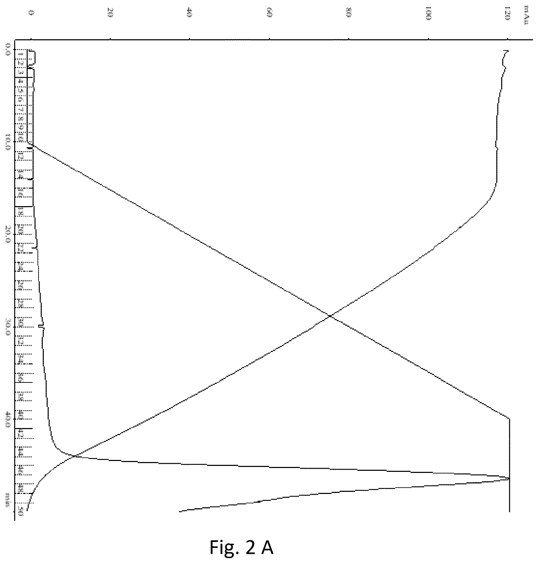

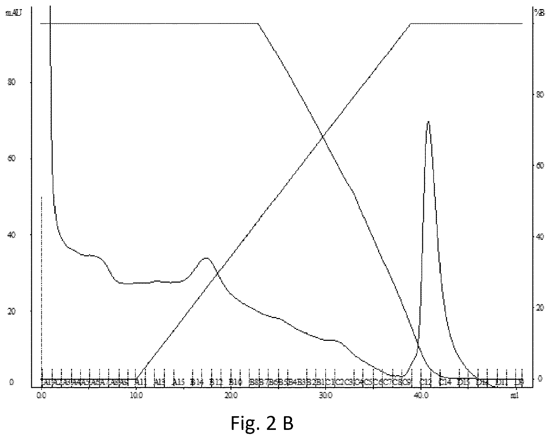

Figure 2. Hydrophobic interaction chromatograms of the antimicrobial peptides secreted by *Lactobacillus alimentarius* MK426 (A) and *Pediococcus acidilactici* MK952 (B). The column was first washed with 50 mM sodium acetate 1.5 M ammonium sulphate (A) and eluted with 50 mM sodium acetate pH 5.6 (B), at a flow rate of 1 mL/min.

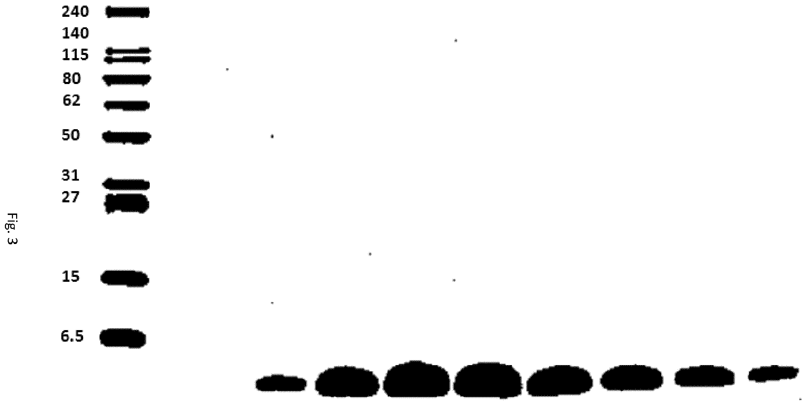

Figure 3. SDS-polyacrylamide gel electrophoresis showing the protein profile of some hydrophobic interaction chromatography fractions eluted with Phenyl HP Column. Eluate aliquots were electrophoresed under denaturing conditions, and Coomassie stained. Lane M: molecular weight marker MultiColour Plus (Grisp); lane 1: HIC flow through; lane 3-7: *Lactobacillus alimentarius* MK426 eluate fractions from HIC column; lane 8-10 *Pediococcus acidilactici* MK952 eluate fractions from HIC column.

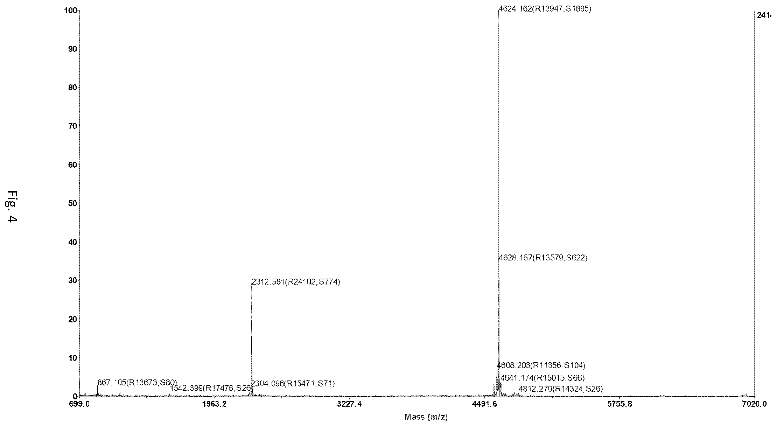

Figure 4. MALDI-TOF mass spectrum of pediocin PA-1.

| m/z | | Intensity | Delta | Modifications | Start | End | Missed | Sequence |
|---|---|---|---|---|---|---|---|---|
| Submitted | Matched | | ppm | | | | Cleavages | |
| 1075.4685 | 1075.4626 | 155.0 | 5.54 | | 12 | 20 | 0 | (K)HSC(Carbamidomethyl)SVDWGK(A) |
| 1246.5981 | 1246.5885 | 1044.0 | 7.75 | | 1 | 11 | 1 | (-)KYYGNGVTC(Carbamidomethyl)GK(H) |
| 2410.1238 | 2410.1139 | 48.0 | 4.10 | | 21 | 43 | 0 | (K)ATCC(Carbamidomethyl)]IINNGAMAWATGGHQGNHK(C) |
| 2426.1145 | 2426.1088 | 296.0 | 2.34 | 1Oxidation | 21 | 43 | 0 | (K)ATCC(Carbamidomethyl)IINNGAMAWATGGHQGNHK(C) |
| 2570.1506 | 2570.1446 | 47.0 | 2.36 | | 21 | 44 | 1 | (K)ATCC(Carbamidomethyl)IINNGAMAWATGGHQGNHKC (Carbamidomethyl)(-) |
| 2586.1448 | 2586.1395 | 219.0 | 2.05 | 1Oxidation | 21 | 44 | 1 | (K)ATCC(Carbamidomethyl)IINNGAMAWATGGHQGNHKC (Carbamidomethyl)(-) |

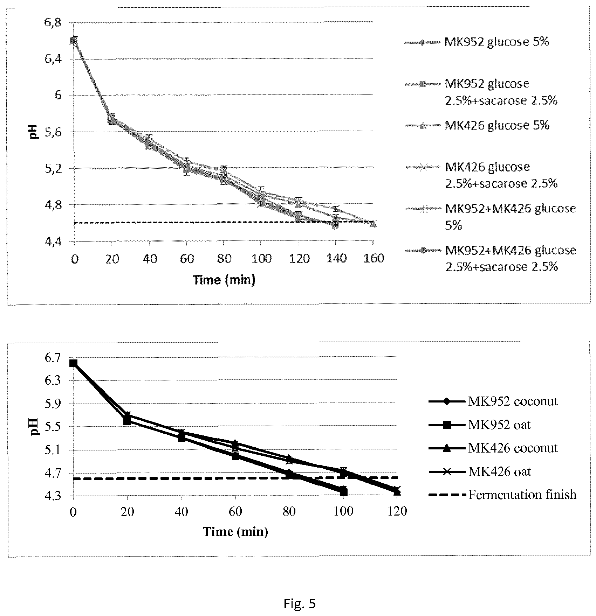

Figure 5. Fermentation profiles during manufacture of the fermented milks (coconut and cow) and oat yogurt-like fermented product.

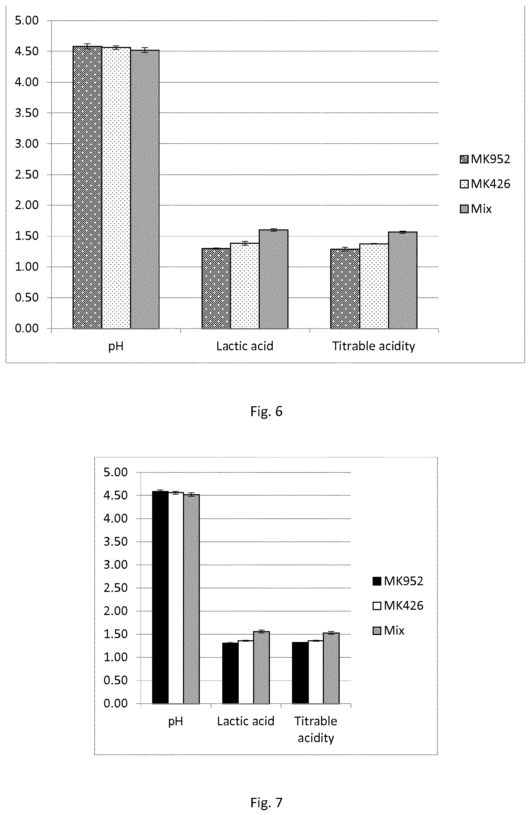

Figure 6. Comparison of parameters related with acidity, between fermented milks (with 5% glucose) fermented by different probiotic strains.

Figure 7. Comparison of parameters related with acidity, between milks (with 2.5% glucose+2.5% sucrose) fermented by different probiotic strains.

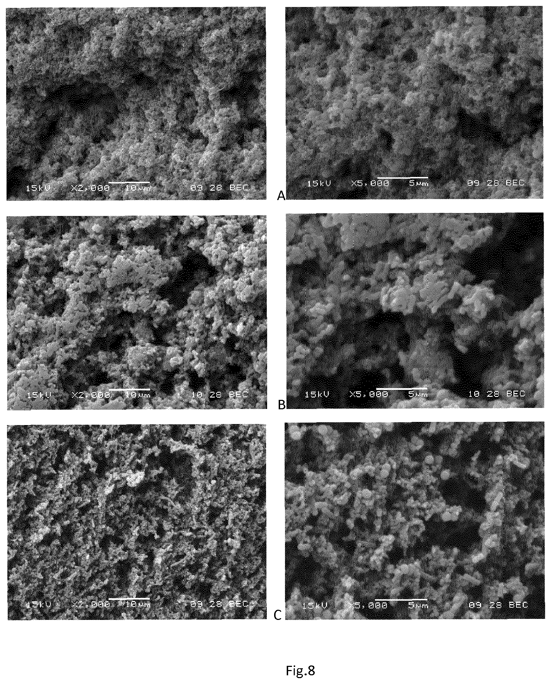

Figure 8. Scanning electron microscope (SEM) micrographs showing the microstructure of milk fermented by *Pediococcus acidilactici* MK952 (A), *Lactobacillus alimentarius* MK426 (B) and co-fermented by both strains (C).

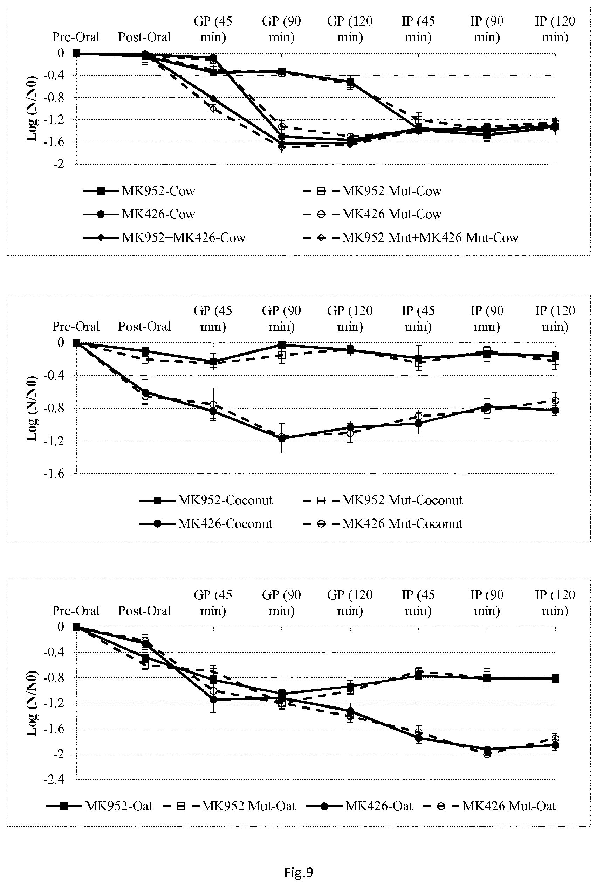

Figure 9. Logarithmic reduction of *Pediococcus acidilactici* strain MK952 (■), *Lactobacillus alimentarius* strain MK426 (•), co-fermented by both strains (♦) and the corresponding isogenic non-bacteriocin-producing mutant strains MK952 mut (□) and MK426 mut (o) and MK952 mut+MK426 mut (◇) during the GIT simulation on dairy, coconut and oat fermented milk/beverage. Each value is the mean ± standard deviation of the mean of three independent experiments.

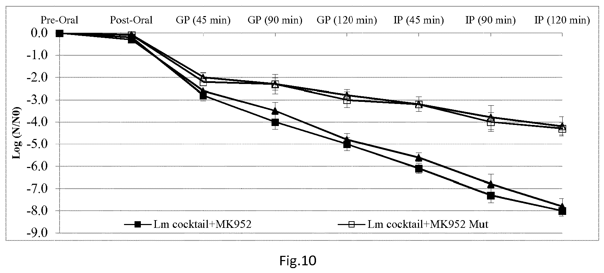

Figure 10. Logarithmic reduction of an eight-strain *Listeria monocytogenes* cocktail inoculated in the milk fermented by *Pediococcus acidilactici* strain MK952 (■), isogenic non-bacteriocin-producing mutant strain MK952 (□), *Lactobacillus alimentarius* strain MK426 (♦) and isogenic non-bacteriocin-producing mutant strain MK952 (◇) during the GIT simulation. Each value is the mean ± standard deviation of the mean of three independent experiments.

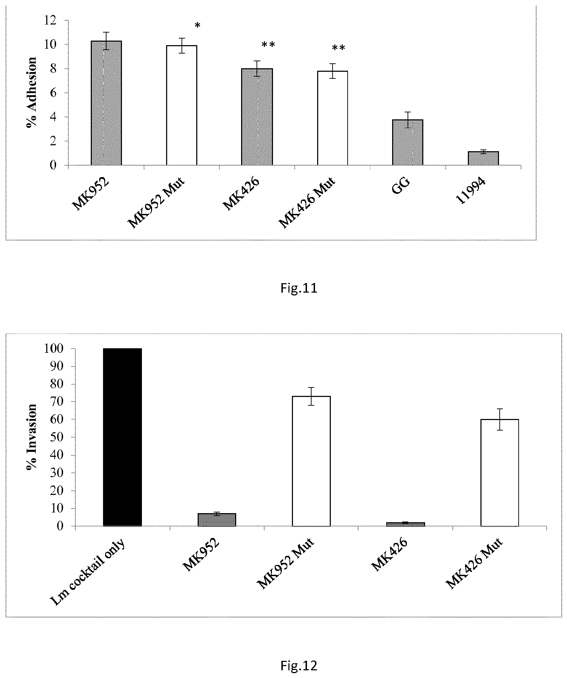

Figure 11. Adhesion profile of the wild-type strains MK952 and MK426, as well as the corresponding isogenic non-bacteriocin-producing mutant strains, MK952 mut and MK426 mut, respectively to the Caco-2 monolayers expressed as the mean ratio (%) between the bacteria recovered from the human cells after incubation and the washing steps and the initial bacterial count of the inoculum (n=2; ± SD).

Figure 12. Invasion of Caco-2 epithelial monolayers by an eight-strain *Listeria monocytogenes* cocktail following pre-treatment of monolayers wild-type strains MK952 and MK426, as well as the corresponding isogenic non-bacteriocin-producing mutant strains, MK952 mut and MK426 mut, respectively (pre-treated monolayers shown by open bars, untreated monolayers shown by solid bars). The marked bars (*) indicate statically significant difference between pre-treated monolayers and untreated monolayers (P<0.05).

## Detailed Description

The present disclosure is also further described, in particular, using embodiments of the disclosure. Therefore, the disclosure is not limited to the descriptions and illustrations provided. These are used so that the disclosure is sufficiently detailed and comprehensive. Moreover, the intention of the drawings is for illustrative purposes and not for the purpose of limitation.

The present disclosure relates to a new bacterium, use of the selected bacterium in a composition, food product or foodstuff comprising the selected bacterium, fermented products and a method to obtain a novel fermented dairy product or dairy alternative product, in particular milk or coconut milk, with probiotic activity.

The present disclosure also relates to a novel fermented dairy product or dairy alternative product, in particular dairy milk or coconut milk comprising the bacterium selected in the present disclosure that may be used to prevent infections, preferably as a prophylactic agent, namely to counteract against *Listeria monocytogenes.*

In an embodiment, an eight-strain *Listeria monocytogenes* cocktail comprising representatives of the most relevant serotypes (1/2a, 1/2b, 4a and 4b) was selected. This approach will ensure a broad assessment of the prophylactic potential of the novel bacteriocinogenic strains, providing a thorough knowledge of the efficacy of the fermented product developed since a robust profile of *Listeria* intrinsic sensibility/resistance to the class IIa bacteriocins, pediocin PA-1 and coagulin, will be evaluated. The *L. monocytogenes* strain ScottA, a clinical isolate recovered from a foodborne outbreak associated with the consumption of pasteurized milk, deposited in the *Listeria* Research Center of Escola Superior de Biotecnologia (LRCESB) was selected. Seven additional strains of *L. monocytogenes* with different origins (recovered from human and food samples), namely strain FSL J1-031 serotype 4a, strains Lm 2542, FSL N3-013, FSL N1-227, CLIP 80459 serotype 4b, strain FSL R2-499 serotype 1/2a and strain FSLJ1-177 serotype 1/2b (listed in table 1) were also included.

In an embodiment, stock cultures of *L. monocytogenes* strains were kept, in tryptic soy broth with 0.6% (w/v) yeast extract (TSBYE, LabM, Bury, UK) supplemented with 30% (v/v) of glycerol (Sigma, Steinheim, Germany), at -80°C. Before use, frozen stocks were aseptically streaked onto brain heart infusion (BHI; Biokar Diagnostic, Beauvais, France) agar plates and incubated at 37°C overnight. Subsequently, one single colony of each *Listeria* isolate was inoculated separately into 5 mL of BHI (Biokar) broth, incubated overnight at 37°C, and subcultured (1% v/v) into 5 mL of BHI broth (Biokar) and incubated in the same conditions.

Table 1 - *Listeria monocytogenes* strains selected for the present study

| Isolate Code | Origin | Sample | Serotype | Isolation Year | Geographic Isolation | Reference |
|---|---|---|---|---|---|---|
| ScottA | Human | Blood | 4b | 1983 | USA | Briers et al. (2011) |
| Lm 2542 | Human | Placenta | 4b | 2010 | Portugal | Magalhães et al. (2015) |
| CLIP 80459 | Human | NA | | | | |
| FSL J1-177 | Human | NA | 1/2b | 1997 | US | De Jesus and Whiting (2003) |
| FSL J1-031 | Human | Blood | 4a | 1991 | Canada | De Jesus and Whiting (2003) |
| FSL N3-013 | Food | Pate | 4b | 1988-99 | UK | Bille and Rocourt (1996) |
| FSL R2-499 | Human | NA | 1/2a | 2000 | US | CDC (2000) |
| FSL N1-227 | Food | NA | 4b | 1988-99 | US | CDC (1998) |

NA= data not available

Commercial probiotic cultures, *Lactobacillus rhamnosus* (Bivos®) and LBA 11994, were grown in *de* Man-Rogosa-Sharpe (Lab M, Bury, UK) broth medium for 18 h at 37°C and subcultured (1% v/v) into 5 mL of MRS broth (Lab M, Bury, UK) and incubated in the same conditions. Stock cultures were kept in MRS broth with 30% (v/v) of glycerol, and stored at - 80°C.

In an embodiment, bacteriocin-producing cultures were isolated. *Lactobacillus alimentarius* MK426 and *Pediococcus acidilactici* MK952 strains were isolated from food matrix. Twenty-five grams of the food sample was added to 225 mL of sterile buffered peptone water, transferred to a stomacher, homogenized for 5 minutes and plated onto de Man-Rogosa-Sharpe agar (MRS, Lab M, Bury, UK) and incubated at 30 °C for 48h, under microaerophilic conditions. Colonies were randomly selected from the plates containing 15 to 150 colonies and cultured in MRS broth for 24h under the abovementioned conditions. The screening for the bacteriocin-producing lactic acid bacteria was performed by the spot-on-lawn soft agar assay. Briefly, 10 $\mu$L of the bacterial cultures were spotted directly onto a BHI soft agar (0.7%) containing the *Listeria* strain ScottA incorporated. Bacteriocin sensitivity was seen as clear zones on the lawn of the indicator target. Positive strains were further characterized with respect to the specific antimicrobial activity according to Tomé *et al* (2006). The positive collected strains were purified by streaking onto de MRS agar and checked through Gram staining and oxidase and catalase activity. Isolates were grown in MRS broth medium for 18 h at 30°C in microaerophilic conditions and stored at -80°C with 30% (v/v) of glycerol. Before use, frozen stocks were aseptically streaked onto MRS agar plates and incubated for 48 h at 30°C in microaerophilic conditions. Subsequently, one single colony of each lactic acid bacteria isolate was inoculated into 5 mL of MRS broth, incubated for 18 h at 30°C, and subcultured twice (1% v/v) into MRS broth and incubated in the same conditions. These strains were identified to genus and species level by means of conventional polymerase chain reaction (PCR) with species- and genus-specific primers, 16S rRNA gene sequencing, as described by Vaz-Moreira et al. (2009).

In an embodiment, the whole genome of both strains was sequenced and assembled. Sequenced reads were demultiplexed automatically by the Illumina® Miseq® sequencer using the CASAVA package (Illumina, San Diego, USA) and quality-filtered with Trimmomatic version 0.30 (Bolger, Lohse, & Usadel, 2014) using the following parameters: 1) bases with average quality lower than Q25 in a window of 5 bases were trimmed, 2) reads with less than 100 bases were discarded and 3) sequencing adapters on reads were removed.

High quality, adapter-free reads were assembled with SPAdes, version 3.9.0 (Bankevich et al., 2012) using the following parameters: -k 21, 33, 55, 77, 99, 127 -careful and --cov- cutoff auto. Assembled contigs with size <500 bp were removed from the assemblies. Assembly metrics were calculated with Quast version 4.6.1 (Gurevich, Saveliev, Vyahhi, & Tesler, 2013). Contigs were checked for contamination and completeness using CheckM 1.0.9 (Parks, Imelfort, Skennerton, Hugenholtz, & Tyson, 2015). Coding gene predictions were made with Prodigal version 2.6 (Hyatt et al., 2010), rRNA and tRNA gene detection using Barrnap version 0.8 (https://github.com/tseemann/barrnap) and CRISPR regions were detected by Minced version 0.2.0 (https://github.com/ctSkennerton/minced). Coding gene annotation has been performed with Prokka version 1.12 (Seemann, 2014) using the following repositories, SwissProt (The UniProt Consortium, 2017), HAMAP (Pedruzzi et al., 2015), TIGRFAMs (Haft, Selengut, & White, 2003) and Pfam (Finn et al., 2016).

Coding genes were also annotated for Pathway using KEGG (Kanehisa, Furumichi, Tanabe, Sato, & Morishima, 2017), for peptidases using MEROPS (Rawlings, Barrett, & Bateman, 2012) and for carbohydrate-active enzymes with dbCaN (Yin et al., 2012).

In an embodiment, bacteriocin activity assay was performed. Bacteriocin sensitivity was determined by a spot-on-lawn soft agar assay. Briefly, 10 $\mu$L of the eluted fractions were spotted directly onto a BHI soft agar (0.7%) containing the *Listeria* strain ScottA incorporated. Bacteriocin sensitivity was seen as clear zones on the lawn of the indicator target. Antilisterial activity of bacteriocins was measured by two-fold dilutions and the spot-on-the-lawn method described by Van Reenen et al. (1998) and expressed in arbitrary unit (AU) mL$^{-1}$. One AU is defined as the reciprocal of the highest dilution showing a clear zone of growth inhibition of indicator bacteria.

In an embodiment, in order to identify the proteinaceous toxins secreted during the late logarithmic and stationary phases of growth of the abovementioned bacteria, a purification protocol was developed. In this sense, the purification of the bacteriocins was performed in a two-step purification methodology comprising ion exchange chromatography (IEX) and hydrophobic interaction chromatography (HIC) for a final polishing to better achieve a high level of purity. Briefly, one litre of MRS broth was inoculated with 1% (v/v) of *Pediococcus acidilactici* MK952 or *Lactobacillus alimentarius* MK426 and incubated for 18 h at 30°C in microaerophilic conditions. Cell free supernatant (CFS) was obtained by centrifuging the culture at 12,000 g for 20 minutes at 4°C. The resulting CFS was then diluted in 1 M of sodium acetate buffer pH 5.6 to a final concentration of 50 mM and vacuum filtered through a 0.22 μm pore size filter, constituting the starting extract for the purification procedures. The resulting diluted sample was applied overnight at 4°C on a 1-mL Hi-Trap Sepharose HP Column (GE Life Sciences) previously equilibrated with 50 mM acetate buffer pH 5.6. Peptide purification was performed on an AKTA Prime Plus system at 4°C. The bounded peptide was then eluted with a linear gradient with an increasing ionic strength up to 1 M NaCl at a flow rate of 1 mL/min. Fractions of 1 mL were collected and assayed for antilisterial activity (spot on lawn and incorporation). The protein profile of the eluted fractions was further analysed by Tricine-SDS-PAGE as described by Schägger (2006). The resolution of proteins was performed in a 4-16% gradient polyacrylamide gel with the Tris/Tricine/SDS buffer system. The peptides were visualized by Coomassie staining and analysed by comparing relative mobility to those of the known molecular weight standard, within the range of 6.5 to 270 kDa (Grisp), run under the same electrophoretic conditions. The most active fractions following the antimicrobial test were polled. The pooled fractions were then submitted to a final polishing step through HIC. Ammonium sulphate was slowly added to the pooled active fractions to a final concentration of 1.5 M avoiding protein precipitation and then loaded on a Phenyl HP Column at a flow rate of 0.5 mL/min. The chromatographic experiment was performed on AKTA Prime Plus system (GE Healthcare) at 4°C. Bacteriocin elution from HIC column occurred over a linear gradient of 1.5 M ammonium sulphate (0% salt- free elution bufffer) to 0 M ammonium sulphate (100% elution buffer) contained within a 50 mM acetate buffer system at a constant pH of 5.6. The antilisterial activity was assessed and the purity of HIC eluate was estimated by SDS-PAGE under denaturing conditions.

In an embodiment, matrix-assisted laser desorption/ionization time-of-flight (MALDI-TOF/TOF) mass spectrometry (MS) measurements were performed to identify the purified peptides. Coomassie-blue stained protein bands were excised from the SDS-polyacrylamide gel, washed, distained and the disulphide bonds reduced with dithiothreitol (DTT). The sample was then alkylated with iodoacetamide (IAA), and digested with trypsin (Promega, USA) following the procedure described by Osório and Reis (2013). Prior to analysis, peptides were acidified using a 2.5% trifluoroacetic acid (TFA) solution, desalted and concentrated using C18 micro-columns (C18 Tips, Thermo Scientific). Tryptic peptides were then crystallized onto a MALDI plate using α-Cyano-4-hydroxycinnamic (8 mg/mL) as a matrix prepared in acetronitrile (50%, v/v), TFA (0.1%, v/v) and 6 mM ammonium phosphate.

Samples were analysed using the 4800 Plus MALDI TOF/TOF Analyzer (AB SCIEX, USA) and peptide mass spectra data were collected in positive ion reflector mode in the range of m/z 700-1,500. All the spectra were externally calibrated using a commercial mixture of angiotensin I, ACTH (adrenocorticotropic hormone) and insulin (AB SCIEX, Framingham, MA) and analysed with the Data Explorer software (Version 4.6, AB SCIEX, Framingham, MA). Peptides were identified by combining Peptide Mass Fingerprint (PMF) with Mascot search engine (Version 2.4, Matrix Science, USA). The peptides identification from acquired MS spectra was performed using a locally stored NCBI copy of protein sequences of the genomes of the lactic acid bacteria previously isolated (*Pediococcus* and *Lactobacillus*). In an embodiment, isogenic non-bacteriocin-producing mutant strains (knockout KO-MK426 and KO-MK952) were generated and used as negative control in the assays herein performed. The plasmid in the mutant strains was cured by subculturing the wild-type bacterial cells in MRS medium supplemented with the aminocoumarin antibiotic, novobiocin, which inhibits DNA gyrase catalysis impairing covalently circular DNA replication. The positive knockout mutant strains were sequenced and confirmed for the loss of pediocin PA-1 and coagulin genes harboured in the corresponding plasmids through plasmid extraction and analysed by electrophoresis.

In an embodiment, a new fermented milk was developed by optimization processes, manufacture and characterization steps.

In an embodiment, an experimental design was conceived in order to optimize cow milk fermentation. The experiment comprised skim and low-fat pasteurized milks; two types of sugar, such as, sucrose and glucose; two hydrocolloids (gelatin and guar gum) in order to increase firmness and viscosity; and skim milk powder to improve the solid matter content. A factorial design was performed with the following concentrations of each mentioned component: sugar (2.5 and 5%, w/v), gelatin (0.4 and 0.6%, w/v), guar gum (0.01%, w/v) and skim milk powder (2, 5 and 10%, w/v). With regard to the dairy alternatives, coconut milk and oat beverage, the experiment comprised low fat coconut milk and oat beverage; one type of sugar, glucose and one hydrocolloid (agar-agar) in order to increase firmness and viscosity. A factorial design was performed with the following concentrations of each mentioned component: sugar (2.5%, w/v) and agar-agar (0.1 and 0.6%, w/v).

In the production process, *Pediococcus acidilactici* MK952, *Lactobacillus alimentarius* MK426 and a co-culture of both, as well as each isogenic non-bacteriocin-producing mutant strains, were introduced into the milk as starter cultures at different concentrations of inoculum (1, 2 and 5%, v/v). LAB strains were grown in three different formulations of a food grade medium, comprising malt sprout (MSM) or barley flour (BFM) or a combination of both (MBFM). Malt sprout (MSM) or barley flour (BFM) (80 g/L) were supplemented with tween 80 (1.08 g/L). Briefly, one single colony of each lactic acid bacteria isolate was inoculated into 5 mL of MSM or BFM broth, incubated for 18h at 30 °C, and subcultured twice (1% v/v) into the same medium. Subsequently, one liter of MSM or BFM was inoculated with a 1% (v/v) inoculum concentration and incubated in the abovementioned conditions, obtaining a final concentration of approximately $10^9$ CFU/mL of each cell suspension. Isogenic non-bacteriocin-producing mutant strains were employed as negative controls for bacteriocin production.

These three different formulations (MSM, BFM and MBFM) will not only ensure a starter culture of vegetal origin to meet the criteria established for the vegetarian fermented milk or beverages, but also a higher antilisterial bacteriocin production by the abovementioned lactic acid bacteria. These novel media will also constitute a valuable strategy to overcome the costly and long fermentation process for large scale production since the inoculum can be directly used from the food grade medium, which also elicit an increment in the fiber content of the milk/beverage.

In an embodiment, the milk was divided in different batches according to the distinct formulations. Sugar, protein solids and/or stabilizers were dispersed under constant homogenization in pasteurized cow milk, coconut milk or oat beverage heated to 80°C. Eighty millilitres of pasteurized milk were poured into 40-mL plastic containers (reduced headspace) and thereafter cooled to the incubation temperature. *P. acidilactici* MK952, *L. alimentarius* MK426 and a co-culture of both were inoculated at the selected concentrations abovementioned. The coagulation of milk was monitored for pH during the incubation period until a pH below 4.6 was reached. When the pH end point was attained, fermented milk was cooled to room temperature and stored at 4°C.

In an embodiment, the fermented milk was characterized. The microbiological analysis was performed. Viable cell counts of the lactic acid bacteria were determined by serial decimal dilutions in sterile Ringer's solution that were subsequently plated on MRS agar medium in duplicate, by the drop count technique. Colonies were enumerated after incubation at 30°C in microaerophilic conditions for 48 h and the CFU/mL values determined.

In an embodiment, chemical composition of the fermented milks was assessed through several different chemical analysis, namely, dry weight, ash, syneresis, fat, protein and titratable acidity. Fatty acids profiles were determined by gas chromatography (GC). High-performance liquid chromatography (HPLC) was also performed, to quantify the amount of sugars, namely, lactose, glucose and sucrose, and citric and lactic acid present in the formulated product.

Dry weight was determined by weighing 5 g of sample to a pre-dried and pre-weighed crucible, and dried at 110°C until constant weight. Ash was obtained by combusting the sample resultant from dry weight determination, at 550°C, for 6 hours. Equations used to calculate moisture and ash were the following:

*Cbef* - Crucible weight before 110°C
*S* - Sample weight
$C_{aft}$ - Crucible weight after 110°C
*Ccomb* - Crucible weight after 550°C

$$Dry\ weight\ (\%) = \frac{Caft - Cbef}{S} \times 100$$

$$Ash\ (\%) = \frac{Ccom - Cbef}{S} \times 100$$

In an embodiment, to quantify syneresis, 5 g of fermented milk were centrifuged, in pre-weighed tubes, at 5,000 rpm for 10 min, at 4°C. Afterwards, supernatant was weighed and syneresis was determined following the equation:

$$Syneresis\ (\%) = \frac{Supernatant\ weight}{Sample\ weight} \times 100$$

In an embodiment, fat content was determined according to Folch (1987), by gravimetry. Five grams were weighed to a centrifuge tube, suspended in 10 mL of chloroform/methanol (2:1, v/v) solvent mixture, vortexed for 15 sec, and sonicated for 30 min. Afterwards, 5 mL of solvent mixture and 5 mL of deionized water were added, the mixture was vigorously shaken for 2 min, and centrifuged at 5,000 rpm for 4 min, at 4°C. Then, the lower phase was transferred to a pre-dried, pre-weighed rotary evaporator balloon. The process was repeated from the addition of the 10 mL of solvent mixture, and the final lower phase was pooled with the first one. The solvent was then evaporated at low temperature (35-40°C), and afterwards the balloon was stored and kept in a desiccator to dry until constant weight was reached, and the final weight was used to calculate the fat content.

Total protein content was assessed through nitrogen determination by Kjeldahl method. Briefly, samples of 200 mg were weighed to Kjeldahl tubes, and 1 g of Missouri catalyst and 4 mL of concentrated $H_2SO_4$ were added to each tube. Samples were digested at 400°C until turning green, and left for another 30 min. Afterwards, tubes were removed from the digestion unit and left to cool down at room temperature and, when cooled, 20 mL of deionized water were added to stop the reaction. Tubes were then transferred to the distillation unit and condensate was collected in Erlenmeyer flask containing 25 mL of boric acid solution, until a volume of 150 mL was reached. Then, titration was performed with HCl 0.1 N, until a turning point of violet. As blank, two tubes with 0.13 g of sucrose were also prepared and simultaneously, and equally, treated.

Nitrogen content was determined according to the following equation:

$$\% \ Nitrogen \ = \ \frac{(mL \ HCl - mL \ blank) \times N \ of \ HCl \times 1.4007}{sample \ weight \ (g)}$$

Protein content was determined by multiplying the obtained nitrogen content by 6.38.

Titratable acidity was determined by weighing 10 g of sample into an Erlenmeyer glass flask, adding 20 mL of deionized water, mixing and adding 4 drops of phenolphthalein. The mixture was then titrated with NaOH 0.1 M, and the volume used in the titration was converted as lactic acid percentage, according to the following equation:

$$\% \ lactic \ acid = \frac{NaOH \ volume \ (L) \times NaOH \ molarity \times 100}{sample \ weight \ (g)} \times Mm(C3H6O3)$$

In an embodiment, in order to quantify the amount of sugars, namely, lactose, glucose and sucrose, and citric and lactic acid present in the fermented milks, HPLC was performed. Two grams of each fermented milk were diluted in 10 mL of sulphuric acid 13 mM, homogenized with an Ultra-Turrax at 18,000 rpm for 3 min and centrifuged at 4,000 rpm for 10 min, at 4°C. The resulting supernatant was then filtered with filter paper no. 1 (V. Reis, Portugal), and immediately prior to injection, with a 0.22 $\mu$m filter. The HPLC system consisted of a Knauer K-1001 pump, a K-2301 RI and K-2501 UV detectors, and an Aminex HPX-87H cation exchange column (300 $\times$ 7.8 mm) (Bio-Rad Laboratories Pty Ltd, Hercules, United States). The mobile phase utilized was 13 mM $H_2SO_4$, delivered at a flow rate of 0.6 mL min$^{-1}$ and the column maintained at 40°C. Peaks were identified by their retention times and quantified using calibration curves prepared with different standards.

In an embodiment, fatty acids profiles were determined by gas chromatography (GC). Duplicate samples of yoghurt were transmethylated, in order to obtain methyl esters of the fatty acids (fatty acid methyl esters; FAME), which were subsequently analyzed by GC. Analysis were performed in duplicate.

One gram was weighed to 14 mL borosilicate glass tubes (16mm $\times$ 125mm) with acid/heat resistant cap (VWR international, Portugal) and 200 $\mu$L of internal standard (IS; glyceryl tritridecanoine, TG-C13; 1.5 mg/mL), and 800 $\mu$L of hexane (Hxn), were added. Then, 2.26 mL of methanol (MetOH) and 240 $\mu$L of sodium methoxide (MetNa; 95%) were also added, and the tubes were vortexed for homogenization. The mixture was heated at 80 ºC for 10 min in a dry-block, and rapidly cooled by placing the tubes on ice. Afterwards, 1.25 mL of dimethylformamide (DMF) and 1.25 mL of sulphuric acid/MetOH (3M) were added, the mixture was vortexed, heated at 60 ºC for 30 min, after which it was again ice cooled. Then, 1 mL of Hxn was added to the mixture, which was vortexed and centrifuged at 1,250 g and 18 ºC, for 5 min. The resulting solution was composed of 3 layers, with an upper hexane layer (containing the FAME), an intermediate layer (interface layer), and a lower methanol layer. The upper layer was collected to be analyzed by GC.

Fatty acid methyl esters were then analyzed in a gas chromatrograph HP6890A (Hewlett-Packard, USA), equipped with a flame-ionization detector and a BPX70 capillary column (60m $\times$ 0.25 mm $\times$ 0.25 $\mu$m, SGE Europe, France). Analysis conditions were as follows: injector (split 25:1; injection volume 1 $\mu$L) and detector temperatures were 250 ºC and 275 ºC, respectively; carrier gas was hydrogen (20.5 psi) and the oven temperature program started at 60 ºC (hold 5 min), raised 15 ºC/min to 165 ºC (hold 1 min), and finally 2 ºC/min to 225 ºC (hold 2 min). Supelco 37 (Sigma-Aldrich, USA) and CRM-164 (Fedelco, Spain) were used for identification of fatty acids. GLC-Nestlé36 (Elysian, USA) was assayed for calculation of response factors and detection and quantification limits (LOD: 0.79 $\mu$g FA/mL; LOQ: 2.64 $\mu$g FA/mL).

In an embodiment, for quantification of the FA the correlation between the concentration and area of the TG-C13 (internal standard) was used, and the same response was assumed for each individual fatty acid.

In an embodiment for better results, one sample of each type of fermented milk was collected and fixed with formaldehyde 37% (v/v) (Merck, Germany), by leaving the sample in contact with the solution for 1 hour. Afterwards, the solution was washed by removing and substituting with deionized water, and leaving for 10 min. Samples were then dehydrated by changing the deionized water with a series of ethanol in increasing concentration (30, 50, 70, 80, 90 and 100% (v/v)). Each dehydration step lasted 10 min. After the 10 min contact with absolute ethanol the solution was removed and hexamethyldisilazane (HMDS; Sigma-Aldrich, United States) has poured over the sample and promptly evaporated with a stream of $N_2$. Dried samples were then placed in observation stubs (covered with double sided adhesive carbon tape (NEM tape, from Nisshin, Japan)) and coated with gold/palladium using a Sputter Coater (Polaron, from Bad Schwalbach, Germany). Samples were analysed in a scanning electron microscope (SEM; JSM-5600LV, from JEOL, Japan) and observations were performed at a working distance of 9 mm, with an acceleration voltage of 15 kV and a spot-size of 28.

In an embodiment, the survival under conditions simulating the human gastrointestinal tract was assessed. The survival of *Pediococcus acidilactici* MK952, *Lactobacillus alimentarius* MK426 and a combination of both, from the formulated fermented milks, alone or challenged with an eight-strain *Listeria monocytogenes* cocktail was evaluated following exposure to a digestion simulation using a static harmonized *in vitro* digestion methodology suitable for food matrices. Isogenic non-bacteriocin-producing mutant strains (knockout (KO-MK426 and KO-MK952) were employed as negative controls. This model describes a three-step procedure mimicking digestive progress in the mouth (oral phase), stomach (gastric phase) and small intestine (intestinal phase), as detailed further below.

With regard to the survival assays performed with the lactic acid bacteria, five grams of the semi-solid fermented milk were transferred into a sterile 50 mL glass flask and immediately exposed to secretions of the oral, gastric and intestinal phases.

In an embodiment, in parallel, *Listeria* strains composing the abovementioned cocktail were grown as previously described, thereafter 1 mL aliquot was centrifuged at 7,000 g for 5 min and the pellet was washed and re-suspended in phosphate buffered saline (PBS, pH=7.4; Sigma-Aldrich St. Louis, MO, USA). One mL aliquots of each cell suspension (~ $10^9$ CFU/mL) were combined and transferred into a sterile 50 mL glass flask containing 5 g of fermented milk.

The mimetization of the oral secretion comprised the addition of simulated salivary fluid (SSF) (KCl 15.1 mmol·L$^{-1}$, KH$_2$PO$_4$ 3.7 mmol·L$^{-1}$, NaHCO$_3$ 13.6 mmol·L$^{-1}$, MgCl$_2$·(H$_2$O)$_6$ 0.15 mmol·L$^{-1}$, (NH$_4$)$_2$·CO$_3$ 0.06 mmol·L$^{-1}$, and HCl 1.1 mmol·L$^{-1}$), CaCl$_2$·(H$_2$O)$_2$ (to obtain 1.5 mmol·L$^{-1}$ in the final mixture) and $\alpha$-amylase in a final concentration of 75 U/mL. The content was thoroughly mixed and the pH of the mixture was measured and if necessary adjusted to pH 7.0 (with and 1M NaOH solution). The samples were then incubated at 37°C in a thermostatic water bath (Julabo SW22, Seelbach, Germany), for two minutes; at this point an 0.1 mL aliquot was collected for bacterial enumeration (post-oral).

The gastric phase encompassed an aqueous acidic secretion simulating the one generated in the stomach compartment, comprising the simulated gastric fluid (SGF) (KCl 6.9 mmol·L$^{-1}$, KH$_2$PO$_4$ 0.9 mmol·L$^{-1}$, NaHCO$_3$ 25 mmol·L$^{-1}$, NaCl 47.2 mmol·L$^{-1}$, MgCl$_2$·(H$_2$O)$_6$ 0.1 mmol·L$^{-1}$, (NH$_4$)$_2$·CO$_3$ 0.5 mmol·L$^{-1}$ and HCl 15.6 mmol·L$^{-1}$), CaCl$_2$·(H$_2$O)$_2$ (to obtain 0.15 mmol·L$^{-1}$ in the fluid) and porcine pepsin solution (2,000 U/mL in the final mixture). The described secretion was added in a ratio of 1:1 (v/v) to each glass flask containing the oral mixture. The samples were homogenised, the pH was adjusted to 3.0 (with a 6 M HCl solution) and incubated in a shaking water bath at 37°C for two hours. For enumeration purposes, 0.1 mL aliquots were collected immediately at time 45, 90, and 120 minutes.

Thereafter, the intestinal phase was mimicked through the addition of simulated intestinal fluid (SIF) (KCl 6.8 mmol.L$^{-1}$, KH$_2$PO$_4$ 0.8 mmol.L$^{-1}$, NaHCO$_3$ 85 mmol·L$^{-1}$, NaCl 38.4 mmol·L$^{-1}$, MgCl$_2$.(H$_2$O)$_6$ 0.33 mmol·L$^{-1}$, and HCl 8.4 mmol·L$^{-1}$), CaCl$_2$·(H$_2$O)$_2$ (to obtain 0.6 mmol·L$^{-1}$ in the fluid), pancreatin from porcine pancreas (in a final concentration of 100 U/mL of trypsin activity) and bile solution (fresh bile in a concentration of 10 mM in the final secretion). The described secretion was added in a ratio of 1:1 (v/v) to each tube containing the gastric mixture. The sample was homogenized, the pH was adjusted to 7 with NaOH 1 M and incubated for 2h at 37°C. Aliquots of 0.1 mL were collected immediately at time 45, 90, and 120 minutes for bacterial enumeration.

For each sampling point decimal dilutions were performed in PBS and plated on MRS agar medium and PALCAM selective agar medium (*L. monocytogenes*), in duplicate, by the drop count technique. Colonies were enumerated after incubation at 37°C for 48 h (*Listeria*) and 30°C for 48 h. *Pediococcus* and *Lactobacillus* CFU/mL values were determined.

In an embodiment for better results, the experiments were performed in duplicate, and the results presented are the mean values of the three independent experiments.

Magnesium chloride hexahydrate, ammonium carbonate, porcine pepsin, pancreatin (8xUSP) and fresh bile were purchased from Sigma-Aldrich (St. Louis, MO), and alpha-amylase was obtained from Megazyme (USA). The remaining reagents were obtained from Merk.

The human epithelial cell line Caco-2 (ATCC HTB-37) originally derived from colorectal tumor was used for *in vitro* assays as a model of the intestinal epithelial barrier. Caco-2 cells were grown and maintained in Dulbecco's minimal essential medium (DMEM) with Earle's salts (Gibco, Gaithersburg, MD, USA) containing 20% fetal bovine serum (Atlanta Biologicals, Inc., Lawrenceville, GA, USA), 1% nonessential amino acids and 1% penicillin-streptomycin at 37°C in a 5% $CO_2$ atmosphere. For adhesion and invasion assays, $4 \times 10^5$ Caco-2 cells were seeded into 24-well plates (Costar, Corning, NY, USA) in DMEM with fetal bovine serum, but without antibiotics and incubated for 48 h at 37°C with 5%

$CO_2$ until confluent.

In an embodiment, Caco-2 monolayers were inoculated with approximately $1\times10^8$ CFU of probiotic bacteria *per* well. Lactic acid bacteria isolates were grown in MRS at 37°C or 30°C, according to the specific strain, for 18 h, without shaking, prior to inoculation. Inoculated Caco-2 monolayers were incubated at 37°C for 2 h. At 120 min post-inoculation, the medium was aspirated and Caco-2 cells were lysed by the addition of 500 μL of ice-cold sterile distilled water and adhered bacteria cells were enumerated by plating on MRS agar plates, which were subsequently incubated at 37°C or 30°C according to the specific strain for 48 h. Two commercial probiotics, *Lactobacillus rhamnosus* (Bivos®) and LBA11994, kindly provided by Christian-Hansen, were also included in each adhesion assay. At least, two independent adhesion assays were performed for each isolate tested, and results were reported as the percentage ratio between the counts of bacteria initially seeded and the counts after the washing steps at the end of the experiment.

In an embodiment, the *in vitro* invasion of the abovementioned eight-strain *Listeria monocytogenes* cocktail was evaluated. The capability of the tested LAB strains to antagonize *in vitro* adhesion and subsequent invasion of the *L. monocytogenes* cocktail was evaluated on Caco-2 cells. Monolayers of Caco-2 cells were inoculated with the abovementioned probiotic strains at a concentration of approximately $1\times10^8$ CFU *per* well. The human cells and LAB were incubated for 2h, followed by three washes with 1 mL sterile phosphate-buffered saline (PBS) to remove any unattached bacterial cell. Caco-2 monolayers were subsequently infected with $1\times10^6$ CFU of *L. monocytogenes* cocktail *per* well. *Listeria monocytogenes* isolates were grown in BHI at 37°C for 18 h, without shaking, prior to inoculation. Plates were then incubated at 37°C with 5% $CO_2$ for 1 h. Infected cells were subsequently incubated for one hour with fresh DMEM with 10% FBS medium containing gentamycin (Gibco) at a concentration of 100 μg/well in order to kill all the extracellular bacteria. The medium was subsequently aspirated and Caco-2 cells were lysed by the addition of 500 μL of ice-cold sterile distilled water and intracellular L. *monocytogenes* cells were serially diluted and enumerated by plating on BHI and PALCAM agar plates, which were subsequently incubated at 37°C for 24 and 48 h, respectively. The results of invasion assay were expressed as percentage ratio of the *L. monocytogenes* recovered from the treated wells and the count from the wells not treated with LAB strains (CFU mL$^{-1}$).

Statistical analysis was performed with IBM SPSS Statistics Version 20 software (IBM, USA). Normality of the data was analyzed by Shapiro-Wilk, and when data was normally distributed analysis of variance (ANOVA), with 95% confidence interval, was applied, in order to assess differences. The Tukey test, with 95% confidence interval, was used for the pair-wise comparisons. When data was not normally distributed Kruskal-Wallis was used to assess differences, and pair-wise comparisons were performed with t-student, with 95% confidence interval.

Two novel bacteriocinogenic lactic acid bacteria were isolated and identified to genus and species level by means of conventional polymerase chain reaction (PCR) with species- and genus-specific primers, 16S rRNA gene sequencing, as *L. alimentarius* MK426 and *P. acidilactici* MK952 and the whole genome sequenced and the corresponding bacteriocins purified and identified.

In an embodiment, bacteriocin purification was analysed (the spot-on-the-lawn assay was performed). A two-step purification procedure composed by cation-exchange and hydrophobic ionic chromatography was developed and generated higher purity peptides, with a final yield of approximately 32%. The first stage, originated a pool of active fractions with an inhibition zone diameter ranging from 2.6-2.9 cm (figure 1). Following the final polishing step through HIC, a single peak was observed, as could be confirmed in the respective chromatogram (figure 2), with the active fractions collected presenting an antilisterial diameter of 3.1 cm. The specific activity of the peptides secreted by *P. acidilactici* MK952 and *L. alimentarius* MK426 was $1.28\times10^6$ and $1.59\times10^5$, respectively. The resolution by SDS-PAGE yielded a unique band of low molecular weight (< 6.5 kDa) for both peptides.

Two peptide bands of interest of each bacteriocin, detected following SDS-PAGE of these fractions were analysed by mass spectrometry (MS/MS), in order to uncover the identity of the purified peptides. The spectrum of the proteinaceous compound, produced by *P. acidilactici* MK952, presented a large number of peptide ion species of significant abundance in the range of *m/z* 700-4,000. The Peptide Mass Fingerprint (PMF) of the tryptic digests, suggested that the matching MH$^+$ peptide mass correspond to all the theorical product ion of the candidate bacteriocin, with the matched peptides covering 100% (44 amino acids/44 amino acids) of the pediocin PA-1 with statistical significance. Additionally, several experimental peptide peaks were also visualized, which could be the result of post-translational modifications. It was confirmed that the proteinaceous compound produced by *P. acidilactici* MK952 was pediocin PA-1 comprising 44 amino acid residues and containing the specific domain YGNGVXC of class IIa bacteriocins.

In an embodiment, with regard to the peptide produced by *L. alimentarius* MK426, the PMF analysis using the MASCOT software has identified with statistical confidence a bacteriocin termed coagulin, which has not been previously reported to be produced by this species. Coagulin, a new member of the pediocin-like family protein, was firstly discovered in *Bacillus coagulans.* Afterward, coagulin was also identified in *Pediococcus pentosaceus* MZF16 (Zommiti et al., 2018).

In an embodiment, a non-conventional cereal-based (malt or barley) growth medium for starter cultures to be employed in the product manufacturing was developed (medium not falling under the scope of protection). Malt sprout, an undervalued and unrecognized by-product solid waste of the brewing industry, as well as barley flour, an agricultural by-product obtained from the barley kernel cutting processes, were used in three different formulations of cereal-based medium. An attempt has been made in order to incorporate these industrial wastes, circumventing the high content of animal-origin elements composing the commercial medium (MRS) ensuring an optimal bacterial growth and bacteriocin production comparable to the MRS. A sustainable approach based on a concept of circular economy was employed, offering an animal free, food grade alternative to the conventional high-cost cultivation media. These three different formulations will not only ensure a starter culture of vegetal origin to meet the criteria established for the manufacturing of vegetarian fermented milk or beverages, but also a higher antilisterial bacteriocin production by the abovementioned lactic acid bacteria. These novel media will also constitute a valuable strategy to overcome the costly and long fermentation process for large scale production since the inoculum can be directly used from the food grade medium, which also elicit an increment in the fiber content of the milk/beverage.

It was initiated the development of the novel product herein proposed formulating a multitude of products on the basis of a multifactorial approach. The obtained fermented milks were then presented in a preliminary and informal sensorial analysis (composed by ten panelists), where a notable acceptance was observed regarding the dairy fermented milk formulated with low fat pasteurized milk and 5% of total sugar (5% of glucose or a combination of 2.5% of glucose and 2.5% of sucrose). Regarding the firmness and texture of the dairy product, this formulation was well accepted, but the addition of gelatin (0.4%) as hydrocolloid in pair combination with skim milk (2%) to increase the total solid content, led to a comparable acceptance and lower syneresis. Concerning dairy alternatives, low fat coconut milk comprising 5% of glucose and 0.25% of agar agar was the preferable combination, whereas the addition of 0.6% of agar agar and 5% of glucose was the most consensual formulation with respect to oat beverage.

According to the results obtained, we sought to deeper characterize the well-accepted fermented milks and plant-based yogurt-like products with the simplest composition, given the low-cost associated with its production and thus high industrial viability and easy scale up. This characterization could serve as a basis to further improve the product, particularly with respect to the firmness and texture.

Incubation temperature is a major factor impacting yogurt properties, as it influences the physical properties, since it conditions acidification and gel formation. Higher incubation temperatures are better suited for the industry, but can have detrimental impact on yogurt characteristics, such as increased whey separation and decreased mechanical properties related with a weaker protein network, such as gel firmness, viscosity and smoothness, which are important sensory properties. In its turn, lower incubation temperatures (32-39 °C instead of 43-45 °C) result in a denser protein matrix, which has a positive impact in the same sensory properties, resulting in yogurts with higher viscosity and smoothness, which are more appealing to consumers.

In the development of this novel product the temperature chosen was 37°C, since it pertained to the abovementioned interval (32-39°C) and the bacterial growth in the milk seemed to be optimum at this temperature in comparison to the 30°C, stated as the suitable temperature for the two lactic acid bacteria of the present disclosure in the novel formulated media. However, this lower range of temperature is also associated with higher production time and costs (Nguyen et al., 2014a).

With respect to the fermented milk microbiological properties, the inoculum used in the present disclosure was composed by *P. acidilactici* MK952 and *L. alimentarius* MK426, alone or in pair combination corresponding to the starter cultures used for fermented milk production (only embodiments in which *L. alimentarius MK426* is employed fall under the scope of protection).

Additionally, the finished product of lactic acid fermentation must contain live lactic acid bacteria (LAB) in amounts $>\_ 7 \log_{10}$ (CFU $g^{-1}$) at the time of manufacture, that should remain active at the end of the stated shelf-life, in order to be considered as a "live and active culture yogurt". Thus, viability analysis to the starter cultures at the end of the fermentative process is preponderant to classify the final product obtained as fermented milk. In addition, the microbiological analysis also provides us information about the starter's growth during fermentation. Therefore, microbial counts of the starter cultures were performed at the beginning and end of fermentation. The starter cultures were inoculated in the milk at a percentage of 2% (v/v), which was stated as the best condition. By the end of the fermentation it was observed an increase of one logarithmic unit consistent for all the strains evaluated, as it presented a similar profile during the homolactic fermentation (Table 2).

Table 2 – Microbiological evaluation of milks fermented by different probiotic strains

*Lactobacillus alimentarius* MK426 (MK426), *Pediococcus acidilactici* MK952 (MK952)

| Milk | Strain | Sugar | Pre-fermentation | Pos-fermentation |
|---|---|---|---|---|
| | | | $\log_{10}$CFU mL$^{-1}$ | $\log_{10}$CFU g$^{-1}$ |
| Cow | | | | |
| | MK952 | $C_6H_{12}O_6$ | $9.22\pm 0.02^a$ | $10.11\pm 0.12^a$ |

| | | | 9.20 ± 0.02[a] | 10.23 ± 0.09[a] |
|---|---|---|---|---|
| | | $C_6H_{12}O_6$ + $C_{12}H_{22}O_{11}$ | | |
| | MK426 | $C_6H_{12}O_6$ | 9.53 ± 0.05[a] | 10.38 ± 0.10[a] |
| | | $C_6H_{12}O_6$ + $C_{12}H_{22}O_{11}$ | 9.51 ± 0.05[a] | 10.35 ± 0.12[a] |
| | Mix | $C_6H_{12}O_6$ | 9.40 ± 0.08[a] | 10.18 ± 0.10[a] |
| | | $C_6H_{12}O_6$ + $C_{12}H_{22}O_{11}$ | 9.44 ± 0.08[a] | 10.18 ± 0.10[a] |
| Coconut | MK952 | $C_6H_{12}O_6$ | 9.41 ± 0.05[a] | 10.28 ± 0.10[a] |
| | MK426 | $C_6H_{12}O_6$ | 9.27 ± 0.06[b] | 10.05 ± 0.08[b] |
| Oat | MK952 | $C_6H_{12}O_6$ | 9.50 ± 0.06[a] | 10.30 ± 0.05[a] |
| | MK426 | $C_6H_{12}O_6$ | 9.19 ± 0.07[b] | 10.08 ± 0.10[b] |

*Note*: Values are average ± standard deviation. Values with the same superscript letter are not significantly different ($p > 0.05$). $C_6H_{12}O_6$ - glucose 5%; $C_6H_{12}O_6$ + $C_{12}H_{22}O_{11}$ - glucose 2.5% + sucrose 2.5%

In an embodiment, with regard to the *in situ* bacteriocin production by the strains of lactic acid bacteria responsible for the milk fermentation detected through phenotypic studies, a significant antilisterial activity was recorded. The recorded values were comparable to the obtained for the commercial medium, which may be related to the secretion of the synthetized intracellular bacteriocins, mediated by the ABC transporters. These results are in agreement with the previously documented by Benkerroum and co-workers (2000) who demonstrated the *in situ* bacteriocin biosynthesis of *Lactococcus lactis* to antagonize *L. monocytogenes* in a traditional fermented milk (Iben). Simova and colleagues (2018) documented the bacteriocinogenic potential of two lactic acid bacteria strains *Lactobacillus bulgaricus* BB18 and *Lactococcus lactis* BCM5, employed as starter cultures in the fermented milk manufacture and presenting a remarkable bacteriocin yield, provided by, according to the authors, the temperature stress which prompted the bacteriocin production.

In an embodiment, fermentation was monitored by pH variation profile (Figure 5), which is one of the most prominent physicochemical parameters in fermented milk manufacturing, since the fermentative process is deemed to be completed when the process of acidification, lowers the pH below the isoelectric point of casein (pH 4.6-4.8). Taking this into consideration, fermented milk was produced at all conditions tested in the present disclosure. In addition, the final pH of the products also gives information about the fermentative rate of each fermentative process, since higher pH values correspond to lower fermentative rates. The results demonstrated a fast-fermentative profile, reaching the desired pH (4.5) in 140-160 min, which is a considerably higher fermentation rate than the usual 240 min (4 hours). Other studies where yogurt fermentation was also evaluated and monitored, like Lee and Lucey (2004), presented a lower fermentation rate (300 min). Sert et al. (2017) investigated the fermentation of a yogurt-like product, which only achieved a pH of 4.5 after 240 min, and Wang (2017), developing a symbiotic corn-based yogurt-like product, also only registered pH of 4.5 at 300 min fermentation. There was a slight difference in the fermentation profile between the strains employed, with *L. alimentarius* MK426 exhibiting a lower fermentation rate (about 20 min more) in comparison with *P. acidilactici* MK952. The globally higher fermentation rate observed for both strains can be due to the glucose added to the milk, as it is a monosaccharide and, therefore, easier to metabolize than the lactose present in the milk, which is a disaccharide composed by glucose and galactose. The fact that lactose is a disaccharide means that the bacteria must first cleave the link between the glucose and galactose units, in order to be able to metabolize the monosaccharides, which takes time, and prolongs the fermentation course.

In an embodiment, results pertaining to chemical composition of the milks fermented by the different probiotic strains (Tables 2 and 3, and Fig. 6 and 7) showed that they were similar. Even though there were, in some parameters, significant differences (p<0.05) between the formulated milks, these were considerably small and as such, overall, the fermented milks can be considered identical.

In an embodiment, the total solids (Table 3) content found in the fermented milks were in agreement with the values reported in other studies performed with commercial yogurts and yogurt-like products.

In an embodiment, concerning ash content (Table 3), although the values can apparently be considered somewhat high, different studies have documented different percentages, varying from 0.26 to 1.4. Olugbuyiro (2011), analysing commercial yogurts, found ash contents ranging from 0.26 to 0.71, while Tarakçi et al. (2003) produced fruit-flavoured yogurts with ash contents as high as 1.1, and Martín-Diana et al. (2003) developed a goat's milk with 1.4. These higher values can be related to higher sugar contents, which can explain the also high values found in the present fermented milks.

In an embodiment, syneresis is one of the most important structural feature of fermented milk, corresponding to the accumulation of whey on its surface. Syneresis percentages (Table 3) were considered a little high when compared with the majority of the values found in literature, which are usually lower (Nguyen et al. 2014). The syneresis values found in the present disclosure, which vary from 30.7 to 31.6 % are similar to the yogurts made from carabao milk documented in Mariano et al. (2011) work, varying from 28.4 to 34.8%, and the fruit flavored yogurts developed by Tarakçi et al. (2003) with 23.23 to 27.20% syneresis. However, there are also milk-type products with high syneresis, such as the yogurts in Sert et al. (2017) study, with values between 47 and 61, and the goat's milk with a syneresis of 63.5. In that paper, a syneresis reduction can be observed with the increase in supplementation with whey protein concentrate (WPC), indicating that higher solid content decreases syneresis. As observed by Nguyen et al. (2014), the protein content may influence the physical properties of yogurt gels, thus, as a consequence, different results may be obtained for different yogurt preparations. In the previous process of the fermented dairy milk optimization we included skim milk (2%) and gelatin (0.4%) to increase the protein content and as such circumvent the syneresis problem.

In an embodiment, protein content is a prominent parameter, and the values obtained concerning dairy fermented milk were between 5.98 and 6.34. These values are significantly higher than those registered by Andleeb et al. (2008) in commercial yogurts (3.09%) and a local commercial yogurt (3.2%). With respect to the plant-based yogurt-like products (coconut and oat), as expected, low protein contents ranging from 0.24-0.26%, in the case of coconut, and 1.38-1.41 % in oat were observed.

In an embodiment, fat is considered a relevant parameter, and the most common fat content in yogurts varies between 0.5 and 3.5%. The fermented dairy milk developed in the present disclosure registered values within those, varying from 1.63 to 1.74%. Olugbuyiro and Oseh (2011) found commercial yogurts with fat content from 1.88 to 4%, and Widodo et al. (2017), working with fermented fresh cow milk, utilizing as starters lactic acid bacteria with intestinal-origin, found fat percentages between 2.20 and 2.56%. Regarding coconut and oat yogurt-like fermented products the values ranged from 7.20-7.49 % and 0.97-1.06 %, respectively.

In an embodiment, titrable acidity is an indirect measure of the amount of lactic acid formed, and is a parameter that can provide information about the extent of the fermentation. Lactic acid is a product of sugar metabolism, being formed during sugars consumption. In the present disclosure there was, as expected, a direct correlation between lactic acid content determined by titrable acidity (Table 3) and lactic acid measured by HPLC (Table 5). Dairy milks fermented by both bacteria, independently, showed similar values, while milks fermented by the mixture presented slightly higher lactic acid content. These values also relate to pH, however, differences in pH (Fig. 6 and 7) were not as accentuated as the ones observed in lactic acid contents. The values obtained herein are somewhat higher than the ones registered in commercial yogurts by Andleeb et al. (2008) (0.84%), and by Sert et al. (2017) in a yogurt-like product (0.84-1.03%), but are similar to the fruit-flavoured yogurts produced by Tarakçi et al. (2003) (1.27-1.36%).

Table 3 – Chemical composition of milks fermented by different probiotic strains,

*Lactobacillus alimentarius* MK426 (MK426); *Pediococcus acidilactici* MK952 (MK952)

| Milk | Strain | Sugar | Total solids | Ash | Syneresis | Fat | Protein | Titratable acidity |
|------|--------|-------|--------------|-----|-----------|-----|---------|--------------------|
| Cow | MK952 | Glu | 83.8 ± 0.1[a] | 0.77 ± 0.01[a] | 31.31 ± 0.72[a] | 1.73 ± 0.01[a] | 6.00 ± 0.23[a] | 1.29 ± 0.03[a] |
| | | Glu + Suc | 83.7 ± 0.1[a] | 0.76 ± 0.02[a] | 30.25 ± 0.68[a] | 1.74 ± 0.02[a] | 5.98 ± 0.18[a] | 1.32 ± 0.01[a] |
| | MK426 | Glu | 83.6 ± 0.1[a] | 0.76 ± 0.01[a] | 30.73 ± 0.80[a] | 1.72 ± 0.02[a] | 6.11 ± 0.15[a] | 1.38 ± 0.01[b] |
| | | Glu + Suc | 83.7 ± 0.1[a] | 0.76 ± 0.01[a] | 31.28 ± 0.60[a] | 1.73 ± 0.02[a] | 6.05 ± 0.20[a] | 1.36 ± 0.02[b] |
| | Mix | Glu | 84.1 ± 0.1[b] | 0.76 ± 0.02[a] | 30.80 ± 0.62[a] | 1.63 ± 0.02[b] | 6.34 ± 0.21[a] | 1.57 ± 0.02[c] |
| | | Glu + Suc | 84.2 ± 0.1[b] | 0.77 ± 0.02[a] | 31.56 ± 0.71[a] | 1.64 ± 0.01[b] | 6.28 ± 0.16[a] | 1.53 ± 0.03[c] |
| Coconut | MK952 | Glu | 82.3 ± 0.1[a] | 0.99 ± 0.04[a] | 32.04 ± 0.69[a] | 7.20 ± 0.02[a] | 0.24 ± 0.12[a] | 3.00 ± 0.15[a] |
| | MK426 | Glu | 86.9 ± 0.1[b] | 0.88 ± 0.04[b] | 28.05 ± 0.82[b] | 7.49 ± 0.03[b] | 0.26 ± 0.16[a] | 4.12 ± 0.23[b] |
| Oat | MK952 | Glu | 81.4 ± 0.1[a] | 1.22 ± 0.12[a] | 33.25 ± 0.65[a] | 1.06 ± 0.01[a] | 1.38 ± 0.10[a] | 3.86 ± 0.22[a] |
| | MK426 | Glu | 84.8 ± 0.1[b] | 0.85 ± 0.01[b] | 29.78 ± 0.72[b] | 0.97 ± 0.02[b] | 1.41 ± 0.14[a] | 4.75 ± 0.38[b] |

Values are percentages and average ± standard deviation. Values with the same superscript letter are not significantly different (p>0.05). Glu - glucose 5%; Glu+Fru - glucose 2.5% + sucrose 2.5%.

Another aim of the present disclosure was to perform an evaluation of the promising strains, *Pediococcus acidilactici* and *Lactobacillus alimentarius* concerning the intrinsic capability to produce or release some of the most prominent fatty acids.

In an embodiment, with respect to coconut milk, it was observed to be an important source of lauric acid (LA) and docosahexaenoic acid (DHA). The encouraging health promoting effects provided by the daily intake of lauric acid, prompted us to analyse the content of this fatty acid and it was found a concentration of nearly 3.6 g/100 g of coconut fermented milk. Lauric acid, a saturated medium-chain fatty acid and the most significant fatty acid of coconut oil, has been reported to positively impact human health, namely due to its notable antimicrobial potential and anti-inflammatory properties, targeting the nuclear factor NF-kB activation and enhancement of proinflammatory cytokines. Lauric acid has been stated to positively hamper breast tumor growth. Indeed, Lappano and coworkers (2017) have conducted an *in vitro* study performed in SkBr3 breast cancer cells and it emerged that LA potentially interfered with the tumour development, triggering the EGFR/ERK signalling pathway, culminating in an impairment of cancer cell proliferation, and prompting cell cycle arrestment and apoptosis. According to the analysis of the fatty acid profile of the novel fermented milk herein presented, it was also observed a docosahexaenoic acid (DHA) production of 14 and 22 mg *per* 100 g of product. Docosahexaenoic acid is a long-chain polyunsaturated omega-3 (n-3) fatty acid that can be found in fish and poultry, and has been known to have a relevant role in the growth and functional development of the brain in infants, as well as being required in adults for the maintenance of normal brain functions. It has also been reported to have a positive effect on senile dementia, by preventing and treating it.

In an embodiment, oat fermented milk proved to be a source of essential fatty acids, namely eicosapentaenoic acid (EPA) (approximately 4mg per 100g), conjugated linoleic acid (CLA) (nearly 7 mg/100g), gamma-linolenic acid (GLA) (4-10 mg/100g), alpha-linolenic acid (ALA) (13-15 mg/100g) and linoleic acid (LNA) (414 and 340 mg *per* 100g).

In an embodiment, eicosapentaenoic acid (EPA) is also a long-chain polyunsaturated omega-3 (n-3) fatty acid, which has been associated with a multitude of health benefits, namely those concerning fetal development, cardiovascular function and Alzheimer's disease. Omega-3 fatty acids, like EPA or DHA, are known to positively impact some neurological and mental conditions, such as depression, and dementia, including Alzheimer's disease, as previously mentioned. Also, beneficial effects in inflammatory conditions have been reported as a consequence of increasing omega-3 fatty acids consumption. Nonetheless, regarding the low biosynthesis capability of the human body to guarantee the proper amount of EPA, this novel fermented milk proved to be a suitable diet supplementation of particular relevance for the pregnant, elderly and patients with a history of coronary artery disease. Besides EPA, as abovementioned, CLA, GLA and LNA are also found in oat fermented milk. These fatty acids also have beneficial health effects. Conjugated linoleic acid (CLA) is a group of linoleic acid isomers (*cis* or *trans*) with a conjugated double bound, which has been found to have anti-obesity, antidiabetic and antihypertensive properties, as well as to have preventive effects on cardiovascular diseases and cancer. Gamma-linolenic acid (GLA) is a polyunsaturated fatty acid with an eighteen-carbon chain, with three *cis* double bonds at positions 6, 9 and 12. It is known to have beneficial effects on aged-linked anomalies, and also to have anti-inflammatory and anticancer activities. Linoleic acid is also a polyunsaturated fatty acid with an eighteen-carbon chain, although with only two double bounds, the first of which at the sixth carbon from the methyl end (carbon 12), making it an omega-6 (n-6) fatty acid, and the other at carbon 9. It is the precursor molecule from which, through a series of reactions (elongation and desaturation), the rest of the n-6 fatty acids can be synthesized. Alpha-linolenic acid (ALA) is another polyunsaturated fatty acid with an eighteen-carbon chain, but with three double bounds, beginning at the third carbon from the methyl end (omega-3). It has the same precursor role regarding n-3 fatty acids, as LNA has in n-6 fatty acids. As they cannot be synthesized *de novo,* LNA and ALA are considered essential fatty acids for mammals. Besides the abovementioned fatty acids, oat fermented milk also possesses positive lipid quality indexes. These indexes are directly correlated with the qualitative aspects of the lipid fraction, and evaluate the lipids predisposition to cause coronary heart disease, being the values inversely correlated. The atherogenic and thrombogenic indexes of this fermented milk are relatively low.

In an embodiment, with respect to the dairy fermented milk, it was observed a gamma linolenic acid (GLA) content of 4.5 mg (MK426), 13.4 mg (MK952) and 18.2 mg (co-culture of MK426 and MK952 strains) *per* 100 g. Conjugated linoleic acid (CLA) was present in a concentration ranging from 0.8 mg (MK426) to 1.7 mg (co-culture) *per* 100 g, which has potential to positively impact health, with antiobesity and antidiabetic properties, as well as preventing cardiovascular diseases and aged-linked anomalies. Besides the abovementioned fatty acids, cow fermented milk also possesses positive lipid quality indexes, presenting relatively low atherogenic and thrombogenic indexes. In this sense the consumption of this novel dairy fermented milk elicits a lower predisposition to coronary heart disease.

Table 4 - Fatty acid profile of milks fermented by different probiotic strains, *Lactobacillus alimentarius* MK426 (MK426); *Pediococcus acidilactici* MK952 (MK952)

| Fatty acid/ Strain | Milk type | | | | | | |
|---|---|---|---|---|---|---|---|
| | Cow | | | Coconut | | Oat | |
| | MK952 | MK426 | MIX | MK952 | MK426 | MK952 | MK426 |
| C4 | 14.322 | 7.344 | 10.429 | 1.578 | 1.444 | 1.231 | 1.264 |
| C6 | 15.937 | 8.419 | 12.242 | 30.324 | 32.151 | <LOD | <LOD |
| C8 | 15.304 | 8.035 | 12.024 | 472.974 | 505.373 | <LOD | <LOD |
| C10 | 39.605 | 21.836 | 30.956 | 433.770 | 463.065 | <LOD | <LOD |
| C10:1 | 3.893 | 2.068 | 3.083 | <LOD | <LOD | <LOD | <LOD |
| C11 | 0.965 | 0.539 | 0.806 | 2.103 | 2.245 | <LOQ | <LOD |
| C12 | 52.705 | 29.570 | 40.925 | 3587.276 | 3768.268 | 1.129 | 1.492 |
| C13i | 1.331 | 0.737 | 1.080 | <LOD | <LOD | <LOD | <LOD |
| C13ai | 0.467 | <LOQ | 0.350 | <LOD | <LOD | <LOD | <LOD |
| C12:1 | 1.395 | 0.775 | 1.106 | <LOD | <LOD | <LOD | <LOD |
| C14i | 1.541 | 0.883 | 1.210 | <LOD | <LOD | <LOD | <LOD |
| C14 | 166.779 | 94.991 | 128.023 | 1292.507 | 1332.974 | 5.290 | 4.447 |
| C15i | <LOQ | <LOQ | <LOQ | <LOD | <LOD | <LOD | <LOD |
| C15ai | <LOQ | <LOQ | <LOQ | <LOD | <LOD | <LOD | <LOD |
| C14:1 | 20.354 | 11.210 | 15.832 | 0.519 | 0.503 | <LOD | <LOD |
| C15 | 7.352 | 4.238 | 5.662 | 0.589 | 0.468 | 1.195 | 0.584 |
| C15:1 | 15.942 | 9.039 | 12.137 | 0.747 | 0.822 | <LOQ | <LOQ |
| C16 | 488.659 | 277.087 | 372.877 | 593.251 | 596.509 | 237.791 | 217.294 |
| C16:1 *t*9 | 1.326 | 0.470 | 1.450 | 0.833 | <LOD | 0.703 | <LOD |
| C16:1 *c*7 | 3.063 | 1.700 | 2.365 | <LOQ | <LOQ | <LOQ | <LOQ |
| C16:1 *c*9 | 19.918 | 14.003 | 20.988 | 3.282 | 1.194 | 4.408 | 2.265 |

(continued)

| Fatty acid/ Strain | Milk type | | | | | | |
|---|---|---|---|---|---|---|---|
| | Cow | | | Coconut | | Oat | |
| | MK952 | MK426 | MIX | MK952 | MK426 | MK952 | MK426 |
| C17i | 9.239 | 2.386 | 3.197 | <LOD | <LOD | <LOD | <LOD |
| C17ai | 11.564 | 4.917 | 6.914 | <LOD | <LOD | <LOD | <LOD |
| C17 | 10.646 | 3.968 | 5.595 | 0.661 | 0.537 | 0.523 | 0.345 |
| C17:1 *c*10 | 8.971 | 1.965 | 2.841 | 0.453 | <LOD | <LOD | <LOD |
| C18i | 0.701 | 0.394 | 0.632 | <LOD | <LOD | <LOD | <LOD |
| C18 | 129.380 | 78.254 | 102.879 | 174.751 | 176.385 | 16.298 | 15.354 |
| C18:1 *t*6-*t*9 | 6.412 | 3.954 | 5.182 | 9.435 | 9.503 | 5.466 | 5.888 |
| C18:1 *t*10 | 16.142 | 10.914 | 14.307 | 2.622 | 2.198 | 2.321 | 2.603 |
| C18:1 *t*11 | 288.874 | 176.262 | 238.305 | 438.920 | 450.782 | 293.540 | 331.848 |
| C18:1 *c*9 | 26.805 | 7.872 | 30.368 | 23.358 | 4.520 | 27.538 | 10.255 |
| C18:1 *t*15 | 5.380 | 3.222 | 3.695 | 1.143 | 0.938 | 0.751 | 0.720 |
| C18:1 *c*11 | 1.331 | 0.762 | 1.083 | 0.318 | <LOQ | 0.516 | 0.483 |
| C18:2 *t*9*t*12 | 3.433 | 2.036 | 2.764 | 0.376 | 0.354 | 1.132 | 1.144 |
| C18:1 *c*14-*t*16 | 3.382 | 2.068 | 2.805 | 0.400 | 0.361 | 1.022 | 1.753 |
| C18:2 *c*9*t*12 | 1.782 | 1.034 | 1.426 | 1.381 | 1.362 | 3.461 | 2.994 |
| C18:2 *t*9*c*12 | <LOD | <LOD | <LOD | 1.309 | 1.232 | 3.006 | 2.681 |
| C18:2 *c*9*c*12 | 34.053 | 18.699 | 27.210 | 89.506 | 90.798 | 413.825 | 340.589 |
| γ C18:3 *c*6*c*9*c*12 | 13.378 | 4.478 | 18.230 | 11.490 | 4.837 | 10.584 | 4.092 |
| α C18:3 *c*9*c*12*c*15 | 3.473 | 1.914 | 2.684 | 0.397 | 0.358 | 15.308 | 12.931 |
| C18:2 *c*9*t*11 | 10.201 | 5.577 | 9.447 | 1.974 | 1.449 | 0.605 | 0.611 |
| CLA *cis c,c*- | <LOD | <LOD | <LOD | 0.335 | 0.312 | 0.188 | 0.273 |
| CLA *cis c,c*- | <LOD | <LOD | <LOD | 2.590 | 2.256 | 5.487 | 6.231 |
| C20:1 *c*9 | 0.862 | 0.712 | 1.025 | 0.315 | <LOQ | <LOD | <LOD |
| CLA *trans t,t*- | 0.834 | 0.457 | 0.967 | 0.373 | 0.308 | 0.267 | 0.335 |
| CLA *trans t,t*- | 0.451 | 0.359 | 0.735 | 0.499 | 0.486 | 0.574 | 0.538 |
| C20:4 n6 | 2.493 | 1.300 | 2.160 | 3.579 | 3.009 | 0.749 | 0.547 |
| C22 | 3.219 | 1.747 | 2.727 | <LOD | <LOD | <LOD | <LOD |
| C20:5 n3 (EPA) | <LOD | <LOD | <LOD | <LOD | <LOD | 3.648 | 3.988 |
| C24 | 3.520 | 2.373 | 6.036 | 5.777 | 10.408 | <LOD | <LOD |
| C22:6 n3 (DHA) | <LOD | <LOD | <LOD | 14.289 | 22.448 | <LOD | <LOD |
| Σ FA | 1467.854 | 831.031 | 1167.212 | 7199.057 | 7490.391 | 1059.221 | 973.917 |

(continued)

| Fatty acid/ Strain | Milk type | | | | | | |
|---|---|---|---|---|---|---|---|
| | Cow | | | Coconut | | Oat | |
| | MK952 | MK426 | MIX | MK952 | MK426 | MK952 | MK426 |
| Σ SFA | 973.707 | 548.181 | 745.015 | 6595.562 | 6889.826 | 263.651 | 240.779 |
| Σ MUFA | 424.021 | 246.740 | 356.516 | 482.542 | 471.356 | 336.736 | 356.183 |
| Σ PUFA | 70.126 | 36.110 | 65.681 | 120.952 | 129.209 | 458.835 | 376.954 |
| Σ CLA | 1.284 | 0.816 | 1.702 | 3.796 | 3.362 | 6.516 | 7.377 |
| Σ ω3 | 3.473 | 1.914 | 2.684 | 14.715 | 14.225 | 18.956 | 16.918 |
| Σ ω6 | 49.925 | 24.477 | 47.600 | 111.437 | 104.954 | 439.274 | 359.425 |
| n6/n3 | 1.435 | 1.279 | 1.771 | 7.540 | 7.750 | 23.174 | 21.241 |
| AI | 0.253 | 0.251 | 0.228 | 15.545 | 16.191 | 0.327 | 0.323 |
| TI | 0.318 | 0.319 | 0.289 | 6.470 | 5.911 | 0.583 | 0.581 |
| HH | 0.014 | 0.011 | 0.017 | 0.072 | 0.069 | 1.951 | 1.717 |

*c = cis* double bound; *t = trans* double bound; <LOQ= below limit of quantification; <LOD = below limit of detection; EPA = eicosapentaenoic acid; DHA = docosahexaenoic acid; Σ = total; FA = fatty acids; SFA = saturated fatty acids; MUFA = monounsatrurated fatty acids; PUFA = polyunsaturated fatty acids; CLA = conjugated linoleic acids; AI = atherogenic index; TI = thrombogenic index; HH = hypocholesterolemic/hypercholesterolemic ratio

Table 5 - Sugars and organic acids present in milks fermented by different probiotic *Lactobacillus alimentarius* MK426 (MK426); *Pediococcus acidilactici* MK952 (MK952)

| Milk | Strain | C source | Lactose | Sucrose | Glucose | Fructose | Citric acid | Lactic acid |
|---|---|---|---|---|---|---|---|---|
| Cow | MK952 | $C_6H_{12}O_6$ | 4.68 ± 0.17[a] | n.a. | 2.11 ± 0.17[a] | n.a. | n.a. | 1.35 ± 0.05[a] |
| | | $C_6H_{12}O_6$ + $C_{12}H_{22}O_{11}$ | 4.85 ± 0.12[a] | 2.41 ± 0.09[a] | 0.02 ± 0.01[b] | n.a. | n.a. | 1.18 ± 0.04[b] |
| | MK426 | $C_6H_{12}O_6$ | 4.78 ± 0.14[a] | n.a. | 2.05 ± 0.13[a] | n.a. | n.a. | 1.38 ± 0.03[a] |
| | | $C_6H_{12}O_6$ + $C_{12}H_{22}O_{11}$ | 4.82 ± 0.11[a] | 2.35 ± 0.12[a] | 0.01 ± 0.01[b] | n.a. | n.a. | 1.21 ± 0.02[b] |
| | Mix | $C_6H_{12}O_6$ | 4.66 ± 0.12[a] | n.a. | 2.17 ± 0.15[a] | n.a. | n.a. | 1.30 ± 0.04[a] |
| | | $C_6H_{12}O_6$ + $C_{12}H_{22}O_{11}$ | 4.74 ± 0.13[a] | 2.38 ± 0.10[3] | 0.02 ± 0.01[b] | n.a. | n.a. | 1.19 ± 0.03[b] |
| Coconut | MK952 | $C_6H_{12}O_6$ (2.5%) | n.a. | 1.25 ± 0.37[a] | 2.21 ± 0.11[a] | n.a. | n.a. | 3.22 ± 0.35[a] |
| | MK426 | $C_6H_{12}O_6$ (2.5%) | n.a. | 0.68 ± 0.09[b] | 2.19 ± 0.01[a] | n.a. | n.a. | 4.66 ± 0.46[b] |
| Oat | MK952 | $C_6H_{12}O_6$ (2.5%) | n.a. | 1.31 ± 0.15[a] | 4.26 ± 0.17[a] | 4.16 ± 0.43[a] | 2.81 ± 0.23 | 4.04 ± 0.52[a] |
| | MK426 | $C_6H_{12}O_6$ (2.5%) | n.a. | 0 | 2.49 ± 0.38[b] | 6.85 ± 0.96[b] | 0 | 5.55 ± 0.21[b] |

Values are expressed in g/100g and are average $\pm$ standard deviation. Values with the same superscript letter are not significantly different ($p$>0.05). $C_6H_{12}O_6$ - glucose 5%; $C_6H_{12}O_6$+$C_{12}H_{22}O_{11}$ - glucose 2.5% + sucrose 2.5%; *n.a. - non applied.*

In an embodiment, sugars and organic acid analysis is presented in Table 5, and showed production of lactic acid concomitant with consumption of sugar, namely, glucose. This is in accordance with the metabolic traits described for the genus of *Pediococcus* and *Lactobacillus,* and particularly *P. acidilactici* and *L. alimentarius,* which have been described as having homofermentative metabolism. This means that the carbon source (sugar) is mainly transformed into lactic acid, via the Embden-Meyerhof-Parnas (EMP) pathway. In the present disclosure results showed that, from the sugar present in the milks, only glucose was consumed, and a 1:1 molar ratio of consumption/production of glucose/lactic acid was observed, which is in accordance with the homofermentative metabolism of the bacteria.

In an embodiment, in order to better understand the impact of the abovementioned probiotics over the fermented milk microstructure, the functional drink was examined through scanning electron microscope (SEM). The correlation between microstructure and texture of fermented milk was analysed, since microstructure has a major impact on the textural and physical properties and also sensory characteristics of fermented milk. Scanning electron micrographs obtained are shown in the figure 8 (A, B and C). It was possible to clearly differentiate the bacillary and coccoid morphologies of the culture microbiota. These bacteria create the desired consistency, taste and flavour of the final product. According to the visualized micrographs, the fermented milk seems to present a uniformly distributed casein network with significant cross-links, which seems to be in good agreement with the results documented by Kristo et al. (2003). The author highlights the impact of the inoculum concentration on the rheological and structural characteristics.

In an embodiment, in order to evaluate the impact of digestion simulation over the new strains of lactic acid bacteria previously isolated, and to assess the antilisterial potential of those during the GIT passage, we analysed the bacterial capability to overcome the harsh gastrointestinal environment, in particular low pH adaptation (stomach) and bile tolerance (upper small intestine). As far as lactic acid bacteria survival is concerned, our results pointed out a notable resistance to the physical stresses encountered following ingestion, with bacteria being able to stably survive. Results pertaining to loss of cell viability throughout GIT transit are shown in Figure 9. In dairy fermented milk, the population of *P. acidilactici* MK952 (9.24$\pm$0.03 $\log_{10}$ CFU g$^{-1}$) and *L. alimentarius* MK426 (initial population of 10.1$\pm$0.06 $\log_{10}$ CFU g$^{-1}$) inoculated alone or co-inoculated (10.2$\pm$0.04 $\log_{10}$ CFU g$^{-1}$) exhibited a slight fluctuation throughout the gastrointestinal exposure, being observed a similar reduction of 1.3 logarithmic units. A similar behaviour was observed for the corresponding isogenic bacteriocin negative mutant strains. Concerning coconut fermented milk, the best results were obtained, with the lowest reductions between the three fermented products. In this milk all strains presented the lowest reduction values. *Pediococcus acidilactici* MK952 and the isogenic non-bacteriocin-producing mutant strain counterpart both presented similar behaviour and only a very slight reduction of 0.2 logaritmic units, while *L. alimentarius* MK426 and the corresponding mutant, which also had similar behaviour between themselves, showed a higher reduction, of 0.7-0.8 logaritmic units. In oat fermented beverage the highest reduction was observed, namely in *L. alimentarius* MK426 and its corresponding isogenic non-bacteriocin-producing mutant strain, with a loss of cell viability of 1.75-1.85 logaritmic units. *Pediococcus acidilactici* MK952 and the mutant isogenic non-bacteriocin-producing strain registered intermediate values, between the three types of fermented products, regarding the decrease throughout GIT, with a reduction of 0.8 logaritmic units. The *in vitro* resistance to the simulated GIT conditions, concomitantly with the conservation of their metabolic activity, is one of the most prominent selection criteria for potential probiotic strains. One relevant and influential factor in the bacterial survival is the protective effect conferred by the food matrix. Fermented milk intrinsic physicochemical characteristics, namely the considerable buffering capacity, partly provided by the caseins and phosphate content, could contribute to minimize the negative impact of the low-pH challenge on the bacterial survival, delaying the acidification thus eliciting the protection of the probiotic strain during the transit through the GIT. Moreover, as described by Kim et al. (1999) and De Angelis et al. (2004) the previous exposure of the lactic acid bacteria to sublethal acidic conditions throughout milk fermentation process, could elicit the development of an adaptive response to acid stress. On the other hand, the composition of the fermented milk, particularly the considerable content of protein and fat could minimize the effect of the enzymes (pepsin, lipase, amylase and proteases) or bile salts on bacterial cells. Furthermore, its semi-solid structured texture could also afford for the observed survival rate. Schillinger and colleagues (2004) also reported the pivotal role conferred by the UHT milk on the lactic acid bacteria survival towards gastric acidity. During the intestinal phase, this protective effect was particularly noticeable for *L. alimentarius* MK426 and the co-culture, abolishing the hostile environment provided by the biological detergent bile. These results are in agreement with the studies previously performed by Jacobsen and colleagues (1999) and Lee and co-workers (2007) which reported the minimal impact of the bile salts on the survival of lactic acid bacteria. On the other hand, it has been documented that some *Lactobacillus,* particularly those isolated from the mammals' gastrointestinal tract present genes encoding the pivotal bile-resistance mechanisms, specifically bile salt hydrolase (BSH) enzymes. In the present disclosure, we observed that viability was maintained up to $10^7$-$10^8$ CFUg$^{-1}$ in fermented milk after a storage at 11 °C and exposure to the intragastric conditions, which is in accordance with the results obtained by Faye and co-workers (2012). Between the three fermented products, coconut fermented milk, as previously mentioned, registered the lowest reduction values and oat fermented beverage the highest, except for *L. alimentarius* MK426 and its corresponding isogenic non-bacteriocin-producing mutant strain. This can be a result of a protective effect conferred by the fat present in the products, as the product with the highest fat content (7.20-7.50 g/100g of product in coconut fermented milk) corresponds to the highest survival, and the product with the lowest fat content (0.97-1.06 g/100g of product in oat fermented beverage) presented the lowest survival (highest reduction).

In an embodiment, these results demonstrated that fermented milk could be considered a suitable vehicle for live microorganisms regarding the notable probiotics ability to tolerate the adverse conditions of the gastrointestinal tract, ensuring that the recommended therapeutic minimum of $10^6$ - $10^7$ colony forming unit (CFU)/g could reach the colon and exert their beneficial effect. In fact, it has been recommended that a probiotic functional food should present live bacteria in the range of $10^8$- $10^9$ CFUg$^{-1}$prior to ingestion.

In an embodiment, in parallel, a multi-strain *L. monocytogenes* cocktail comprising the most prominent serotypes (clinical and food isolates) were selected to evaluate *Listeria* survival when intragastric inoculated in the novel formulated dairy fermented milk through simulated gastrointestinal tract conditions. Results are presented in Figure 10.

In an embodiment, the initial population of *L. monocytogenes* intragastric inoculated during the dairy fermented milk digestion with wild-type *P. acidilactici* MK952 (initial population of 9.24±0.03 log$_{10}$ CFU g$^{-1}$) and *L. alimentarius* MK426 (initial population of 10.2±0.04 log$_{10}$ CFU) was 8.03±0.05 log$_{10}$ CFU g$^{-1}$. Pasteurized milk has been associated with outbreaks of listeriosis in which the estimated ingested dose was significantly low (<$10^4$). The subsequent passage through the gastrointestinal tract lead to a negligenciable reduction during the oral phase and a prominent reduction in the gastric phase, in which *L. monocytogenes* viable counts decreased to values below the detection limit of the enumeration method. The direct incorporation of live bacteriocin producing-strains positively protected the host gastrointestinal tract from this opportunistic pathogen, exerting concomitantly a pivotal role in impeding *Listeria* infection. In a parallel study conducted with each isogenic non-bacteriocin-producing mutant strains challenged with the abovementioned *Listeria* cocktail, we observed an extraordinary survival rate of this pathogen to the simulation of the gastrointestinal conditions. In fact, this result is explained by the absence of the antilisterial bacteriocin production, along with the pathogen capacity to develop acid tolerance on the basis of the complex arsenal of genes encoding proteins of the arginine deiminase (ADI) acid tolerance system and glutamate decarboxylase (GAD) system. Intriguingly, factors that may be associated with the gastrointestinal tract, namely the low pH, anaerobiosis, hypo and hyperosmotic shock, bile salts and chloride ions, have been shown to induce GAD system expression in a variety of bacteria. On the other hand, it has also been documented *Listeria* amazing capability to colonize the lumen of the gallbladder, through particular mechanisms of resistance, namely Sigma B, BSH and BilE. In this sense, the observed *Listeria* reduction, when intra-intestinal inoculated in the digestive process of the dairy fermented milk containing the wild-type strains was attributed to the bacteriocinogenic potential of the lactic acid bacteria incorporated. Indeed, a remarkable antilisterial effect was observed following exposure to simulated gastric conditions, in which viable cell counts of *L. monocytogenes* decreased to values below the detection limit of the enumeration method. Given the *Listeria* intrinsic resistance to low pH, it is feasible to conclude that the mechanism by which the probiotic bacteria antagonized the pathogen was through *in situ* (gut) bacteriocin production, which could be a notable mean to overcome the susceptibility of these peptides to some gastrointestinal proteases. Indeed, Kheadr and co-workers (2010) performed an *in vitro* dynamic human gastrointestinal model study to evaluate the efficacy of *in situ* pediocin PA-1 delivery. It was found that *Pediococcus acidilactici* UL5 recovered from the gastric, duodenal, jejunal and ileal compartments retained the potent bacteriocinogenic activity against *Listeria ivanovii*. The results presented in Figure 10 and the corresponding discussion concern only to dairy fermented milk, which serves as an example, since the behavior of all the strains (including the corresponding isogenic non-bacteriocin-producing mutant strains) was similar in all the three fermented products.

In an embodiment, the ability of lactic acid bacteria to adhere to mucus and human intestinal epithelial cells has been suggested as a crucial feature for their colonization and persistence in the GIT, being considered a relevant criterion for the evaluation of the probiotic potential of LAB. The mechanism by which probiotics may adhere to the intestinal mucin and enterocytes, following the initial attachment to the epithelium surface, encompasses pivotal interactions between bacterial adhesins and epithelial receptors. In this sense, in order to assess the adhesion capability of the new promising bacteriocin-producing strains (*L. alimentarius* MK426 and *P. acidilactici* MK952), we performed adhesion assays on monolayers of the epithelial Caco-2 cells. This intestinal cell line has been extensively used to mimic the small intestinal epithelium, since they may differentiate into enterocytes and thus could be a suitable model to indirectly evaluate the lactic acid bacteria ability to colonize the intestinal tract. Focusing attention on the adhesion profile of the two novel putative probiotic strains, it was observed that the new strains MK952 and MK426 presented higher adhesive potential in comparison with the two commercial probiotics included in the present disclosure. Despite the fact that adhesion is referred as a strain specific feature, MK952 and MK426 presented a similar attachment profile. *Pediococcus acidilactici* MK952 exhibited the highest adhesiveness percentage of approximately 10.3%, and the corresponding isogenic non-bacteriocin-producing mutant strain presented a similar adherence value of 9.9%. These values are slightly higher than the findings of Palencia and co-workers (2009) who reported adherence values of 6.1% with respect to *P. parvulus* 2.6 and with the documented by Koo and colleagues (2012) who observed that *P. acidilactici* presented an adherence percentage ranging from 4.5-6%. With respect to *L. alimentarius* MK426, and the corresponding mutant strain, exhibited an adhesion of 8 % and 7.8%, which is within the range of previously documented values. The observed profile is also in agreement with the study conducted by Koo and co-workers (2012) who registered adhesion percentages of 11.8% for *L. casei* and 10.2% for *L. paracasei.* In the cited study, low adherence percentages were also documented for *L. acidophilus* with a value of 4%. This value is in accordance with the obtained for the commercial probiotics *L. rhamnosus* and LBA 11994 which demonstrated low adhesion ability, presenting adhesiveness values of 3.75 and 1.11%, respectively. However, it is noteworthy that probiotics may positively impact host health without colonization of the gastrointestinal tract.

In an embodiment, the pathogenicity of *L. monocytogenes* relies on its ability to attach to and invade the gastrointestinal epithelium and subsequently withstand the host immune response. The ability of probiotic LAB strains to prevent *L. monocytogenes* infection has been demonstrated by both cell culture and animal model studies. The mechanisms by which potentially probiotic strains might antagonize *L. monocytogenes* have been widely studied. A multitude of mechanisms have been proposed to explain the capability of probiotic LAB to counteract the effects of *L. monocytogenes* in the gastrointestinal tract; among which might be cited, the competitive exclusion of the pathogen, through blocking its contact with receptors expressed on epithelial cells and subsequent downstream effects (Corr et al., 2009), the potential to directly exert their inhibitory activity through the secretion of proteinaceous metabolites, which may act alone or synergistically. In the present disclosure, approximately $1 \times 10^6$ CFUmL$^{-1}$ of an eight-strain *Listeria monocytogenes* cocktail invaded untreated Caco-2 monolayers. *Listeria monocytogenes* cocktail comprising representatives of the most relevant serotypes (1/2a, 1/2b, 4a and 4b) presented a prominent decreased invasiveness of epithelial monolayers subsequently to the pre-exposure of epithelial monolayers to the wild-type assayed bacteria. It was observed that the infection of *L. monocytogenes* strain tested was significantly reduced to 7 % with regard to the wild-type strain MK952, while the corresponding isogenic mutant strain (MK952 mut) barely impaired *Listeria* infection, which presented an invasion value of 73%. On the other hand, the strain MK426 exhibited a notable potential in eliciting a reduction of 98% in relative invasion efficiency of *L. monocytogenes* cocktail. A dissimilar behaviour was observed for the isogenic mutant strain in comparison with the wild-type strain MK426, which only promoted a decrease of 40% in *Listeria* invasiveness. The findings herein presented pointed out that there is not a correlation between probiotics adhesion ability and their antagonist effect toward *Listeria* invasion, corroborating the previous studies of Koo et al. (2012) and Botes et al. (2008). In fact, despite the significant adherence of *P. acidilactici* MK952, the bacteria exhibited the lowest potential (93%) to effectively inhibit *Listeria* invasion. On the basis of these results, one can conclude that the almost complete abolishment of *Listeria* cocktail invasiveness recorded, provided by the novel bacteriocinogenic probiotics was the result of the *in situ* secretion of antilisterial proteinaceous metabolites (bacteriocins) by the strains MK952 and MK426, pediocin PA-1 and coagulin, respectively. In fact, when exposed to the corresponding isogenic non-bacteriocin-producing mutant strains, the multistrain *Listeria* cocktail presented a notable virulence, detaining a significant infection capability. Indeed, this explanation is in agreement with the previously reported by Corr and co-workers (2007) who demonstrated that some strains of *Lactobacillus* presented a prominent anti-invasive activity toward *Listeria* due to the secretion of proteinaceous compounds, excluding the mode of action reliant on the competitive exclusion. The authors highlighted that these molecules might promote the secretion of mucin. These results were substantiated by Lim and Im (2012), who documented that *Lactobacillus* free supernatants can impede *Listeria* adhesion to intestinal cells.

In an embodiment, the rationale of the employment of a *L. monocytogenes* cocktail comprising eight representatives of the most relevant serotypes (1/2a, 1/2b, 4a and 4b), displaying a panoply of different profiles of resistance/sensibility to pediocin PA-1 and coagulin substantiated the prophylactic potential of these novel probiotic strains, given their notable capability to produce each bacteriocin in the gut. In this sense, the efficacy of the novel fermented products herein presented to counteract a multistrain *Listeria* cocktail was clearly demonstrated regardless of the virulence/resistance of the *Listeria* strain.

Elderly represents the fastest growing class of the population in the Western world. Advances in the field of medicine are leading to growing life expectancies of population, and to a longer endurance of immunocompromised and elderly individuals, which are at higher risk for listeriosis. Furthermore, a revolution of social and food habits is taking place worldwide with an increasing demand for ready-to-eat processed foods that represent the most threat for *L. monocytogenes* infection. The invasion of the intestinal barrier is the first step in the *L. monocytogenes* infection mechanism, thus probiotics may provide significant protection against listeriosis. In this sense, the use of probiotics may represent a new effective tool to effectively counteract this pathogen with regard to intestinal colonization and organ invasion.

In the present disclosure a novel functional probiotic-based fermented product was developed, in particular milk or vegetable yogurt-like products, and the results surprisingly show the potential of *in situ* antilisterial bacteriocin production to be used as prophylactic therapy for *L. monocytogenes* infection. In fact, the results demonstrated that dairy and coconut fermented milk, as well as oat fermented beverage could be considered suitable vehicles for the delivery of live microorganisms regarding the notable ability of *P. acidilactici* MK952 and *L. alimentarius* MK426 to tolerate the harsh conditions of the gastrointestinal tract. In fact, both bacteria stably survive the passage through the human GI tract when administered orally in the formulated fermented milk/ yogurt-like product, ensuring that the recommended therapeutic minimum of $10^6$ - $10^7$ colony forming units (CFU)/g could reach the colon and exert their beneficial effect. Indeed, it has been recommended that a probiotic functional food should present live bacteria in the range of $10^8$- $10^9$ CFUg$^{-1}$ prior to ingestion. According to our experimental data, the high bacteriocin and probiotic cell concentration in the formulated fermented milk proved to exert efficient bactericidal effect on *Listeria* in the gastrointestinal tract during milk intake.

Overall, the results regarding the adhesion and invasion assays pointed out that *L. alimentarius* MK426 and *P. acidilactici* MK952 presented strong adhesive features and remarkable ability to inhibit Listeria infection. Indeed, the wild-type strains MK426 and MK952, coagulin and pediocin PA-1 producers, exhibited a notable potential in eliciting an almost abolishment in relative invasion efficiency of *L. monocytogenes* cocktail. A dissimilar behaviour was observed for the isogenic coagulin and pediocin PA-1 negative mutant strains, which were incapable of preventing *Listeria* invasiveness and concomitantly *Listeria* infection, in the same extent of the wild-type strains. Concerning our results, we proved in a multistrain *Listeria* monocytogenes cocktail, comprising representatives of the most relevant serotypes, detaining a large spectrum of intrinsic sensibility/resistance to coagulin and pediocin PA-1, a notable impairment of *Listeria monocytogenes* infection elicited by the gut coagulin delivery (*in situ*) secreted by the novel probiotic strains *L. alimentarius* MK426 and *P. acidilactici* MK952, which we previously isolated (whole genome sequenced; bacteriocins purified and identified). By generating bacteriocin-negative mutant strains, failing to produce coagulin and pediocin PA-1, we observed no efficacy in the impairment of *Listeria* invasion, unveiling the pivotal role of the coagulin and pediocin PA-1 (class lla) in mediating the observed antagonism exerted by the novel probiotics on the multistrain *L. monocytogenes* cocktail. These findings support the rationale that pediocin PA-1 and coagulin directly exert a bactericidal effect, targeting *Listeria* through the membrane Man-PTS system. These probiotic strains proved to be suitable for the development of these new functional foods, helpful in counteracting *Listeria* infection. Furthermore, regarding the almost complete impairment of *L. monocytogenes* cocktail invasiveness, both strains proved to be promising candidates for *in vivo* animal and human trials to validate the notable *in vitro* results. Blocking the initial adhesion/invasion of *L. monocytogenes* provided by the ingestion of the formulated milk would be a rational strategy for controlling infection. Also disclosed (not falling under the scope of protection) is a non-conventional cereal-based (malt or barley) growth medium for starter cultures to be employed in the product manufacturing. Malt sprout, an undervalued and unrecognized by-product solid waste of the brewing industry, as well as barley flour, an agricultural by-product obtained from the barley kernel cutting processes, were used in three different formulations of cereal-based medium. An attempt has been made in order to incorporate these industrial wastes, circumventing the high content of animal-origin elements composing the commercial medium (MRS) ensuring an optimal bacterial growth and bacteriocin production comparable to the MRS. A sustainable approach based on a concept of circular economy was employed, offering an animal free, food grade alternative to the conventional high-cost cultivation media. These three different media formulations, MSM, BFM and MBFM, will not only ensure a starter culture of vegetal origin to meet the criteria established for the manufacturing of vegetarian fermented milk or beverages, but also a higher antilisterial bacteriocin production by the abovementioned lactic acid bacteria. These novel media will also constitute a valuable strategy to overcome the costly and long fermentation process for large scale production since the inoculum can be directly used from the food grade medium, which also elicit an increment in the fiber content of the milk/beverage. Only Examples in which*L. alimentarius MK426* is employed are Examples according to the present invention.

## EXAMPLE 1 - COW MILK

Dissimilar to Actimel, in which *L. casei* is added *post* fermentation, the novel probiotic bacteria replace the conventional starter cultures, presenting a notable fermentation capability and the final product contains ten thousand million bacteria *per* g of product with bacteriocinogenic potential to counteract *Listeria monocytogenes*. A natural source of vitamin B12, gluten-free, presenting a high protein content (6 g) and a source of gamma-linolenic acid (GLA) and conjugated linoleic acid (CLA), which has potential to positively impact health, with antiobesity and antidiabetic properties, as well as preventing cardiovascular diseases and aged-linked anomalies. Besides the abovementioned fatty acids, cow fermented milk also possesses positive lipid quality indexes, presenting low atherogenic and thrombogenic indexes. In this sense elicits a lower predisposition to coronary heart disease.

## EXAMPLE 2 - COCONUT MILK

In an embodiment, a novel plant-based fermented milk was tested namely coconut milk, lactose-free, source of fiber with prophylactic potential against *Listeria.* Both strains presented a notable capability to withstand the harsh conditions of the gastrointestinal tract, achieving the intestine in concentrations of $10^9$ CFU/mL. It is an important source of ascorbic acid presenting 2.8 mg of vitamin C (20% of the daily recommended dose) and minerals, namely potassium (250 mg), phosphorous (100 mg), manganese (1mg), magnesium (37 mg) (6%, 14%, 44% and 10% of the daily recommended intake (DRI), respectively). With respect to the essential fatty acids content present in the coconut milk, it was observed to be an important source of lauric acid (LA) and docosahexaenoic acid (DHA). The encouraging health promoting effects provided by the daily intake of lauric acid, prompted us to analyse the content of this fatty acid and it was found a concentration of nearly 3.6 g/100 g of coconut fermented milk. Lauric acid (LA), a saturated medium-chain fatty acid and the most significant fatty acid of coconut oil, has been reported to positively impact human health, namely due to its notable antimicrobial potential and anti-inflammatory properties, targeting the nuclear factor NF-kB activation and enhancement of proinflammatory cytokines. Lauric acid has been stated to positively hamper breast tumor growth.

**EXAMPLE 3** - **OAT MILK**

In an embodiment, a novel plant-based yogurt-like product with prophylactic potential regarding listeriosis, having oat beverage as milk replacer. Cholesterol free, lactose free, comprising almost 0% of unhealthy saturated fat, 3 g of total fiber, termed beta-glucan, detaining a hypocholesterolemic capability, lowering the serum cholesterol and as such preventing the risk of cardiovascular disease. Oat fermented milk proved to be a source of essential fatty acids, namely eicosapentaenoic acid (EPA) (approximately 4 mg *per* 100g), conjugated linoleic acid (CLA) (nearly 7 mg/100g), gamma-linolenic acid (GLA) (10 and 4 mg/100g), alpha-linolenic acid (ALA) (13-15 mg/100g) and linoleic acid (LNA) (414 and 340 mg *per* 100g).

Eicosapentaenoic acid (EPA) is also a long-chain polyunsaturated omega-3 (n-3) fatty acid, which has been associated with a multitude of health benefits, namely those concerning fetal development, cardiovascular function and Alzheimer's disease. Omega-3 fatty acids, like EPA or DHA, are known to positively impact some neurological and mental conditions, such as depression, and dementia, including Alzheimer's disease, as previously mentioned. Also, beneficial effects in inflammatory conditions have been reported as a consequence of increasing omega-3 fatty acids consumption. Nonetheless, regarding the low biosynthesis capability of the human body to guarantee the proper amount of EPA, this novel fermented milk proved to be a suitable diet supplementation of particular relevance for the pregnant, elderly and patients with a history of coronary artery disease. Besides EPA, as abovementioned, CLA, GLA and LNA are also found in oat fermented milk. These fatty acids also have beneficial health effects. Conjugated linoleic acid is a group of linoleic acid isomers (*cis* or *trans*) with a conjugated double bound, that has been found to have anti-obesity, antidiabetic and antihypertensive properties, as well as to have preventive effects on cardiovascular diseases and cancer. Gamma-linolenic acid is a polyunsaturated fatty acid with an eighteen-carbon chain, with three *cis* double bonds at positions 6, 9 and 12. It is known to have beneficial effects on aged-linked anomalies, and also to have anti-inflammatory and anticancer activities. Linoleic acid is also a polyunsaturated fatty acid with an eighteen-carbon chain, although with only two double bounds, the first of which at the sixth carbon from the methyl end (carbon 12), making it a omega-6 (n-6) fatty acid, and the other at carbon 9. It is the precursor molecule from which, through a series of reactions (elongation and desaturation), the rest of the n-6 fatty acids can be synthesized. Alpha-linolenic acid is another polyunsaturated fatty acid with an eighteen-carbon chain, but with three double bounds, beginning at the third carbon from the methyl end (omega-3). It has the same precursor role regarding n-3 fatty acids, as LNA has in n-6 fatty acids. As they cannot be synthesized de *novo,* LNA and ALA are considered essential fatty acids for mammals. Besides the abovementioned fatty acids, oat fermented milk also possesses positive lipid quality indexes. These indexes are directly correlated with the qualitative aspects of the lipid fraction, and evaluate the lipids predisposition to cause coronary heart disease, being the values inversely correlated. The atherogenic and thrombogenic indexes of this fermented milk are relatively low.

**EXAMPLE 4 (coagulin)**

According to PMF analysis using the MASCOT software we have identified with complete statistical confidence that a bacteriocin termed coagulin was produced by the novel strain *L. alimentarius* MK426, which has not been previously reported to be produced by this species. Coagulin, a new member of the pediocin-like family protein, was firstly discovered in *Bacillus coagulans.* Afterward, coagulin was also identified in *Pediococcus pentosaceus* MZF16 (Zommiti et al., 2018). The wild-type strain MK426, coagulin producer exhibited a notable potential in eliciting a reduction of 98% in relative invasion efficiency of *L. monocytogenes* cocktail. A dissimilar behaviour was observed for the isogenic coagulin negative mutant strain, which were incapable of preventing *Listeria* invasiveness and concomitantly *Listeria* infection, in the same extent of the wild-type strains. Concerning our results, we proved in a multistrain *Listeria* monocytogenes cocktail, comprising representatives of the most relevant serotypes, detaining a large spectrum of intrinsic sensibility/resistance to coagulin, a notable impairment of *Listeria monocytogenes* infection elicited by the gut coagulin delivery (*in situ*) secreted by the novel probiotic strain *L. alimentarius* MK426, which we previously isolated (whole genome sequenced; bacteriocins purified and identified). By generating bacteriocin-negative mutant strains, failing to produce coagulin, we observed no efficacy in the impairment of *Listeria* invasion, unveiling the pivotal role of the coagulin (class IIa) in mediating the observed antagonism exerted by the novel probiotics on the multistrain *L. monocytogenes* cocktail. These findings support the rationale that coagulin exerts directly its bactericidal effect, targeting *Listeria* through the membrane Man-PTS system and not through another mechanism. Indeed, the results demonstrated that dairy and coconut fermented milk, as well as oat fermented yogurt-like fermented product could be considered suitable vehicles for the delivery of these live microorganisms regarding the notable ability of *L. alimentarius* MK426 and its bacteriocin coagulin, to overcome the harsh conditions of the gastrointestinal tract, namely the low pH adaptation and (stomach) and bile tolerance (small intestine). The notable coagulin tolerance (when protected by the fermented milk/beverage) to the gastric enzymes, pepsine, likewise the significant resistance to pancreatin (amylase, lipase and protease) afforded to the prominent protection against *Listeria* infection.

The term "comprising" whenever used in this document is intended to indicate the presence of stated features, integers, steps, components, but not to preclude the presence or addition of one or more other features, integers, steps, components or groups thereof.

It will be appreciated by those of ordinary skill in the art that unless otherwise indicated herein, the particular sequence of steps described is illustrative only and can be varied without departing from the disclosure. Thus, unless otherwise stated the steps described are so unordered meaning that, when possible, the steps can be performed in any convenient or desirable order.

The disclosure should not be seen in any way restricted to the embodiments described and a person with ordinary skill in the art will foresee many possibilities to modifications thereof.

The above described embodiments are combinable.

The following claims further set out particular embodiments of the disclosure.

The following documents are referred to:

Corr, S.C., C. Hill, and C.G. Gahan. 2009. "Advances in Food and Nutrition Research 56." In Press, Academic.

Dobson, Alleson, Paul D. Cotter, R. Paul Ross, and Colin Hill. 2012. "Bacteriocin Production: A Probiotic Trait?" Applied and Environmental Microbiology 78(1): 1-6. Lee and Lucey (2004) Structure and Physical Properties of Yogurt Gels: Effect of Inoculation Rate and Incubation Temperature.

Nguyen (2014) The Effect of Fermentation Temperature on the Microstructure, Physicochemical and Rheological Properties of Probiotic Buffalo Yoghurt.

Radoshevich, Lilliana, and Pascale Cossart. 2018. "Listeria Monocytogenes: Towards a Complete Picture of Its Physiology and Pathogenesis." Nature Reviews Microbiology 16(1): 32-46. http://dx.doi.org/10.1038/nrmicro.2017.126.

## Claims

1. *Lactobacillus alimentarius* MK426 with a complete genome sequence of: SEQ ID NO 1 for use in prevention of *Listeria* infection.

2. *Lactobacillus alimentarius* MK426 with a complete genome sequence of: SEQ ID NO 1 for use as a probiotic agent or as a nutraceutical agent.

3. Use of *Lactobacillus alimentarius* MK426 as described in claim 1 for fermented food products preparation.

4. Composition comprising *Lactobacillus alimentarius* MK426 as described in claim 1.

5. Composition according to the previous claim for use in prevention of *Listeria* infection or as a probiotic.

6. Composition or composition for use according to any of the previous claims wherein the composition is a food composition or a pharmaceutical composition.

7. Composition or composition for use according to any of the previous claims wherein the composition is dried, lyophilized or in paste form.

8. Composition or composition for use according to any of the previous claims further comprising carbohydrates, hydrocolloids, skim milk powders, stabilizers, emulsifiers, preservatives, buffers, or mixtures thereof.

9. Composition or composition for use according to any of the previous claims, wherein the number of bacteria varies from 8-12 log CFU mL$^{-1}$, preferably 10.1-10.4 log CFU mL$^{-1}$.

10. Food product or foodstuff comprising *Lactobacillus alimentarius* MK426 as described in claim 1.

**11.** Food product or foodstuff according to the previous claim, wherein the food product or foodstuff is a diary product or dairy alternative product comprising a bacteriocin obtainable by the fermentation with *Lactobacillus alimentarius* MK426.

**12.** Food product or foodstuff according to any of the previous claims 10-11, wherein the product is milk, cheese, yogurt, frozen yogurt, or ice-cream, an oat derivate, a coconut derivate, a soy derivate or mixtures thereof.

**13.** Method of preparing starter cultures of *Lactobacillus alimentarius* MK426 as described in claim 1 using cereal-based culture medium as growth medium for starter culture, wherein the cereal-based culture medium comprises malt sprout and barley flour.

**14.** Method for obtaining a dairy product or dairy alternative product comprising the steps of:

fermenting the dairy product or dairy alternative product by *Lactobacillus alimentarius* MK426 as described in claim 1;
in particular wherein the amount of said *Lactobacillus alimentarius* vary from 0.5 to 2% (v/v);
in particular wherein the fermentation time varies from 140 to 160 min;
in particular wherein the pH varies from 4.4 to 4.6.

**Patentansprüche**

**1.** *Lactobacillus alimentarius* MK426 mit einer vollständigen Genomsequenz von: SEQ ID NO 1 zur Verwendung bei der Prävention von Infektionen mit *Listeria.*

**2.** *Lactobacillus alimentarius* MK426 mit einer vollständigen Genomsequenz von: SEQ ID NO 1 zur Verwendung als probiotisches Mittel oder als nutrazeutisches Mittel.

**3.** Verwendung von *Lactobacillus alimentarius* MK426, wie in Anspruch 1 beschrieben, zur Herstellung fermentierter Lebensmittel.

**4.** Zusammensetzung, umfassend *Lactobacillus alimentarius* MK426, wie in Anspruch 1 beschrieben.

**5.** Zusammensetzung nach dem vorangehenden Anspruch zur Verwendung bei der Prävention von Infektionen mit *Listeria* oder als ein Probiotikum.

**6.** Zusammensetzung oder Zusammensetzung zur Verwendung nach einem der vorangehenden Ansprüche, wobei die Zusammensetzung eine Nahrungsmittelzusammensetzung oder pharmazeutische Zusammensetzung ist.

**7.** Zusammensetzung oder Zusammensetzung zur Verwendung nach einem der vorangehenden Ansprüche, wobei die Zusammensetzung in getrockneter, gefriergetrockneter oder in pastöser Form vorliegt.

**8.** Zusammensetzung oder Zusammensetzung zur Verwendung nach einem der vorangehenden Ansprüche, ferner umfassend Kohlenhydrate, Hydrokolloide, Magermilchpulver, Stabilisatoren, Emulgatoren, Konservierungsmittel, Puffer oder Mischungen davon.

**9.** Zusammensetzung oder Zusammensetzung zur Verwendung nach einem der vorangehenden Ansprüche, wobei die Anzahl der Bakterien von 8-12 log KbE mL$^{-1}$, bevorzugt 10,1-10,4 log KbE mL$^{-1}$ variiert.

**10.** Lebensmittelprodukt oder Lebensmittel, umfassend *Lactobacillus alimentarius* MK426 wie in Anspruch 1 beschrieben.

**11.** Lebensmittelprodukt oder Lebensmittel nach dem vorangehenden Anspruch, wobei das Lebensmittelprodukt oder Lebensmittel ein Milchprodukt oder ein Milchalternativprodukt ist, umfassend ein Bakteriocin, das durch die Fermentation mit *Lactobacillus alimentarius* MK426 erhältlich ist.

**12.** Lebensmittelprodukt oder Lebensmittel nach einem der vorangehenden Ansprüche 10-11, wobei das Produkt Milch, Käse, Joghurt, gefrorener Joghurt oder Speiseeis, ein Haferderivat, ein Kokosnussderivat, ein Sojaderivat oder Mischungen davon ist.

13. Verfahren zur Herstellung von Starterkulturen von *Lactobacillus alimentarius* MK426, wie in Anspruch 1 beschrieben, unter Verwendung eines Kulturmediums auf Getreidebasis als Wachstumsmedium für die Starterkultur, wobei das Kulturmedium auf Getreidebasis Malzkeime und Gerstenmehl umfasst.

14. Verfahren zur Herstellung eines Milchprodukts oder Milchalternativprodukts, umfassend die Schritte: Fermentieren des Milchprodukts oder Milchalternativprodukts durch *Lactobacillus alimentarius* MK426, wie in Anspruch 1 beschrieben;

wobei insbesondere die Menge des genannten *Lactobacillus alimentarius* von 0,5 bis 2 % (v/v) variiert;
wobei insbesondere die Fermentationsdauer zwischen 140 und 160 Min. variiert;
wobei insbesondere der pH-Wert zwischen 4,4 und 4,6 variiert.

## Revendications

1. *Lactobacillus alimentarius* MK426 avec une séquence du génome complète de : SEQ ID NO 1 destiné à être utilisé dans la prévention d'une infection de *Listeria.*

2. *Lactobacillus alimentarius* MK426 avec une séquence du génome complète de : SEQ ID NO 1 destiné à être utilisé en tant qu'agent probiotique ou en tant qu'agent nutraceutique.

3. Utilisation de *Lactobacillus alimentarius* MK426 tel que décrit dans la revendication 1 pour la préparation de produits alimentaires fermentés.

4. Composition comprenant le *Lactobacillus alimentarius* MK426 tel que décrit dans la revendication 1.

5. Composition selon la revendication précédente destinée à être utilisée dans la prévention de l'infection de *Listeria* ou en tant que probiotique.

6. Composition ou composition destinée à être utilisée selon l'une quelconque des revendications précédentes dans laquelle la composition est une composition alimentaire ou une composition pharmaceutique.

7. Composition ou composition destinée à être utilisée selon l'une quelconque des revendications précédentes dans laquelle la composition est séchée, lyophilisée ou sous forme de pâte.

8. Composition ou composition destinée à être utilisée selon l'une quelconque des revendications précédentes comprenant également des glucides, hydrocolloïdes, poudres de lait écrémé, stabilisants, émulsifiants, conservateurs, tampons, ou des mélanges de ceux-ci.

9. Composition ou composition destinée à être utilisée selon l'une quelconque des revendications précédentes, dans laquelle le nombre de bactéries varie de 8 à 12 log UFC mL$^{-1}$, préférablement de 10,1 à 10,4 log UFC mL$^{-1}$.

10. Produit alimentaire ou denrée comprenant le *Lactobacillus alimentarius* MK426 tel que décrit dans la revendication 1.

11. Produit alimentaire ou denrée selon la revendication précédente, dans lequel le produit alimentaire ou denrée est un produit laitier ou une alternative de produit laitier comprenant une bactériocine pouvant être obtenue à travers la fermentation avec le *Lactobacillus alimentarius* MK426.

12. Produit alimentaire ou denrée selon l'une quelconque des revendications précédentes 10-11, dans lequel le produit est du lait, fromage, yaourt, yaourt glacé, ou crème glacée, un dérivé d'avoine, un dérivé de noix de coco, un dérivé de soja ou des mélanges de ceux-ci.

13. Procédé de préparation de ferments de *Lactobacillus alimentarius* MK426 tel que décrit dans la revendication 1 utilisant un milieu de culture à base de céréales en tant que milieu de croissance de ferments, dans lequel le milieu de culture à base de céréales comprend des germes de malt et de la farine d'orge.

**14.** Procédé d'obtention d'un produit laitier ou d'une alternative de produit laitier comprenant les étapes consistant à :

fermenter le produit laitier ou l'alternative de produit laitier à l'aide du *Lactobacillus alimentarius* MK426 tel que décrit dans la revendication 1 ;
en particulier dans lequel la quantité dudit *Lactobacillus alimentarius* varie de 0,5% à 2% (v/v) ;
en particulier dans lequel le temps de fermentation varie de 140 à 160 min ;
en particulier dans lequel le pH varie de 4,4 à 4,6.

Fig. 1 A

Fig. 1 B

Fig. 2 A

Fig. 2 B

Fig. 3

Fig. 4

Fig. 5

Fig. 6

Fig. 7

Fig.8

Fig.9

Fig.10

Fig.11

Fig.12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **YANXIN HU et al.** Novel bacteriocin products by Lactobacillus alimentarius FM-MM 4 from a traditional Chinese fermented meat Nanx Wudl: Purification, identification and antimicrobial characteristics. *Food Control.*, 06 February 2017, vol. 77, 290-297
- **JUVEN BENJAMIN J et al.** Growth and Survival of Listeria monocytogenes in Vacuum-Packaged Ground Beef Inoculated with Lactobacillus alimentarius FloraCarn L-2. *Journal of Food Protection,* 1998, vol. 61, 551-556
- **CORR, S.C. ; C. HILL ; C.G. GAHAN.** Advances in Food and Nutrition Research 56. In Press, Academic, 2009

- **DOBSON, ALLESON ; PAUL D. COTTER ; R. PAUL ROSS ; COLIN HILL.** Bacteriocin Production: A Probiotic Trait?. *Applied and Environmental Microbiology,* 2012, vol. 78 (1), 1-6
- **LEE ; LUCEY.** *Structure and Physical Properties of Yogurt Gels: Effect of Inoculation Rate and Incubation Temperature,* 2004
- **NGUYEN.** The Effect of Fermentation Temperature on the Microstructure. *Physicochemical and Rheological Properties of Probiotic Buffalo Yoghurt,* 2014
- **RADOSHEVICH, LILLIANA ; PASCALE COSSART.** Listeria Monocytogenes: Towards a Complete Picture of Its Physiology and Pathogenesis. *Nature Reviews Microbiology,* 2018, vol. 16 (1), 32-46, http://dx.doi.org/10.1038/nrmicro.2017.126